# EUROPEAN PATENT SPECIFICATION

(11) **EP 3 938 378 B1**
(45) Date of publication and mention of the grant of the patent: **02.08.2023**
(21) Application number: 20708137.3
(22) Date of filing: 11.03.2020
(51) Int. Cl.: C07K 14/08, C12N 15/40, C12N 7/00, A61K 39/12

(54) **NOROVIRUS-LIKE PARTICLES WITH IMPROVED STABILITY**
NOROVIRUS-ÄHNLICHE PARTIKEL MIT VERBESSERTER STABILITÄT
PARTICULES DE TYPE NOROVIRUS À STABILITÉ AMÉLIORÉE

(30) Priority: 12.03.2019 EP 19162266
(43) Date of publication of application: 19.01.2022
(73) Proprietor: Icon Genetics GmbH, 06120 Halle (Saale) (DE)
(72) Inventor: JARCZOWSKI, Franziska, 06317 Seegebiet Mansfelder Land (DE); DIESSNER, André, 06122 Halle (Saale) (DE); THIEME, Frank, 06317 Seegebiet Mansfelder Land (Ortsteil Lüttchendorf) (DE)
(74) Representative: Blodig, Wolfgang
(86) International application number: PCT/EP2020/056461
(87) International publication number: WO 2020/182860

(56) References cited:
- EP-A1- 3 153 578
- WO-A1-2018/170603
- Y. SOMEYA ET AL: "Assembly of homogeneous norovirus-like particles accomplished by amino acid substitution", JOURNAL OF GENERAL VIROLOGY., vol. 92, no. 10, 29 June 2011 (2011-06-29) , pages 2320-2323, XP055599285, GB ISSN: 0022-1317, DOI: 10.1099/vir.0.033985-0 cited in the application
- K. DEBBINK ET AL: "The State of Norovirus Vaccines", CLINICAL INFECTIOUS DISEASES, vol. 58, no. 12, 27 February 2014 (2014-02-27), pages 1746-1752, XP055306273, US ISSN: 1058-4838, DOI: 10.1093/cid/ciu120

## Description

### FIELD OF THE INVENTION

The present invention provides a Norovirus (NoV) genogroup I VP1 capsid protein that is capable of forming a VLP (virus-like particle). The invention further provides a VLP containing said capsid protein, and an immunogenic composition and a vaccine containing said capsid protein or said VLP. The invention also relates to a nucleic acid molecule encoding the capsid protein. The invention further provides an immunogenic composition and a vaccine containing said capsid protein or said VLP and a genogroup II or genogroup IV capsid protein or VLP. The invention further provides the capsid protein, the VLP, or the vaccine for use in the prevention or treatment of Norovirus infection in a subject, preferably a human. Also provided is a method of increasing the stability of Norovirus genogroup I VLPs.

### BACKGROUND OF THE INVENTION

Noroviruses are the leading cause of gastroenteritis outbreaks worldwide. They are responsible for 685 million cases annually including 200 million cases among children 5 years old or younger (www.cdc.gov/norovirus/worldwide.html). Up to date, there is no norovirus vaccine on the market. Also, there are no established protocols for norovirus cultivation, which significantly slows down progress with norovirus vaccine development. In addition, the rapid rate of the genetic changes of circulating noroviruses leads to new norovirus strains emerging every 2-4 years, causing epidemic outbreaks and complicating the development of vaccines and therapies that are required to counter these challenges (de Graaf, M, van Beek, J, & Koopmans, PG, 2016, Nature Rev Microbiol, 14: 421-433). It is evident that genogroup G! and GII representatives have been the main causes of the majority of outbreaks in the last two decades (Matthews et al., 2012, Epidemiol. Infect, 140: 1161-1172) with prevalence of the GII genogroup genotype 4 (GII.4). For example, since 2014, appearance of new GII.17 strains has been described in East Asia as well as re-emergence of old GII.4 strains (Chan et al, 2015, Nat Commun, doi: 10.1038/ncomms10061; Choi et al, 2017, Food Environ Virol, doi: 10.1007/s12560-017-9278-4). This constantly changing landscape adds complexity to defining an efficient vaccine composition, as a generally preferred approach is the use of a multivalent vaccine. The current norovirus vaccine development mainly relies on the use of virus-like particles (VLPs) vaccines (for recent reviews please see: Tan, M. & Jiang, X., 2014, Hum. Vaccin Immunother, 10:1449-1456; Debbink, K., Lindesmith, L. & Baric, R.S., 2014, Clin Infect Dis, 58:1746-1752; Ramani, S., Estes, M.K. & Atmar, R.L., 2016, PLoS Pathog, 12:e1005334) predominantly produced in animal cell culture, specifically in insect cells using baculoviral expression system (Huhti, L., et al., 2013, Arch. Virol., 158: 933-942; Koho, T., et al., 2012, J. Virol. Methods, 181: 6-11).

A major issue for development of polyvalent vaccines is that even genetically relatively closely related Norovirus isolates exhibit distinct VLP stability profiles (Pogan, R, et al., 2018, Curr Opin Virol, in press; Pogan, R, et al., 2018 J. Phys.: Condens. Matter, 30:064006). Therefore, preparation of multivalent vaccines that consist of a mix of different VLPs which require different buffer conditions due to their different stability profiles is a serious challenge for formulation. In some cases, this problem is solved by adding alum as stabilizer and adjuvant to the VLP mix (Leroux-Roels, G., et al., 2018, The J. Infect. Diseases, 217:597-607). However, despite the fact that alum-containing vaccines are generally well-tolerated, alum has long-lasting biopersistence in the body, ability to migrate in lymphoid organs and accumulate in brain, which raises concerns especially for pediatric applications (Petrovsky, N. & Aguilar, JC., 2004, Immunol. &Cell Biol., 82:488-496; Gherardi R.K., et al., 2014, Front. Neurol, 6:4; Gherardi R.K., et al, 2016, Morphologie, 100:85-94). An alternative approach is amino acid substitution of critical residues leading to more homogeneous VLP (Someya, Y., et al., 2011, Journal of General Virology, 92:2320-2323) and could also improve stability of VLPs (Pogan, R., et al., 2018, J. Phys.: Condens. Matter, 30:064006) even in a more complex formulation of a VLP mixture.

Departing from the prior art, it is an object of the present invention to provide Norovirus VLPs, notably VLPs of genogroup I, of improved stability and capsid proteins for such VLPs. It is another object to provide bivalent or multivalent immunogenic compositions and vaccines that address the issue of compatibility and stability of Norovirus VLPs to allow formulation of vaccine compositions without the need of stabilizers like alum. Therefore, it is also an object to provide compositions comprising NoV VLPs of different genogroups having comparable or similar stability. It is another object to provide protocols for purification, preparation and formulation of VLPs, notably of VLPs of different genogroups such as genogroups I and II.

### SUMMARY OF THE INVENTION

These objects are solved by:
1. A norovirus genogroup I VP1 capsid protein, wherein the amino acid sequence of said protein has at the position corresponding to Arg472 in SEQ ID NO:3 an Arg or Lys residue.
2. A norovirus genogroup I VP1 capsid protein having an amino acid sequence wherein the amino acid sequence stretch Ala-Ala-Leu-Leu/Val-His-Tyr (SEQ ID NO: 57) is modified to Ala-Ala-Leu-Leu/Val-Arg/Lys-Tyr (SEQ ID NO: 58), wherein Leu/Val means either Leu or Val, and Arg/Lys means either Arg or Lys.
3. The capsid protein according to item 2, wherein the amino acid sequence stretch Ala-Ala-Leu-Leu/Val-His-Tyr-Val/Leu/lle-Asp (SEQ ID NO: 59) is modified to Ala-Ala-Leu-Leu/Val-Arg/Lys-Tyr-Val/Leu/lle-Asp (SEQ ID NO: 60), wherein Val/Leu/lle means either Val or Leu or Ile.
4. The capsid protein according to any one of items 1 to 3, wherein said capsid protein belongs to any of the following genotypes of genogroup I: GI.1, GI.2, GI.3, GI.4, GI.5, GI.6, GI.7, GI.8, or GI.9.
5. The capsid protein according to any one of items 1 to 4, wherein said capsid protein has an amino acid sequence as defined in any one of SEQ ID NOs: 6 to 21, except that the amino acid residue at the position corresponding to Arg472 in SEQ ID NO:3 is Arg or Lys; or said protein has an amino acid sequence as defined in any one of SEQ ID NOs: 3 to 5.
6. The capsid protein according to any one of items 1 to 5, wherein
   (i) the amino acid sequence of said protein consists of or comprises at least 480, preferably at least 500 contiguous amino acid residues of the amino acid sequence of any one of SEQ ID NOs: 6 to 21, except that the amino acid residue at the position corresponding to Arg472 in SEQ ID NO:3 is Arg or Lys; or
   (ii) the amino acid sequence of said protein consists of or comprises at least 480, preferably at least 500 amino acid residues and has at least 80%, preferably at least 85%, more preferably at least 90% sequence identity over this length to a sequence segment of at least 500 contiguous amino acid residues of the amino acid sequence of any one of SEQ ID NOs: 6 to 21; or
   (iii) the amino acid sequence of said protein has from 1 to 50, preferably from 1 to 40, more preferably from 1 to 30 deletions, substitutions, additions or insertions compared to a sequence segment of at least 480, preferably at least 500 contiguous amino acid residues of the amino acid sequence of any one of SEQ ID NOs: 6 to 21;
   whereby in items (ii) or (iii) the amino acid residue at the position corresponding to Arg472 in SEQ ID NO:3 is Arg or Lys.
7. The capsid protein according to any one of items 1 to 6, wherein
   (i') the amino acid sequence of said protein consists of or comprises the amino acid sequence from residue 43 to residue 540 of SEQ ID NO:3, 4, or 5; or
   (ii') the amino acid sequence of said protein has at least 80 %, preferably at least 85%, more preferably at least 90% sequence identity to the amino acid sequence from residue 43 to residue 540 of SEQ ID NO: 3, 4, or 5; or
   (iii') the amino acid sequence of said protein has from 1 to 50, preferably from 1 to 40, more preferably from 1 to 30 deletions, substitutions, additions or insertions compared to the amino acid sequence from residue 43 to residue 540 of SEQ ID NO: 3, 4, or 5;
   whereby in items (ii') or (iii') the amino acid at the position corresponding to Arg472 in SEQ ID NO:3, or at the position corresponding to His472 in SEQ ID NO:2, is Arg or Lys.
8. Nucleic acid molecule encoding the capsid protein according to any one of items 1 to 7.
9. Virus-like particle (VLP) consisting of or comprising the capsid protein according to any one of items 1 to 7.
10. An immunogenic composition comprising the capsid protein as defined in any one of items 1 to 7 or the VLP of item 9 and optionally a pharmaceutically acceptable carrier.
11. The immunogenic composition according to item 10, further comprising a Norovirus genogroup II VP1 capsid protein, or a VLP consisting of or comprising a genogroup II VP1 capsid protein.
12. A vaccine against norovirus infection, comprising the immunogenic composition of item 10 or 11.
13. A vaccine against norovirus infection, comprising the capsid protein as defined in any one of items 1 to 7 or the VLP of items 9.
14. The vaccine according to item 12 or 13, comprising a Norovirus genogroup II VP1 capsid protein or a VLP consisting of or comprising a genogroup II VP1 capsid protein.
15. The capsid protein as defined in any one of items 1 to 7, or the VLP of item 9, or the vaccine according to any one of items 12 to 14 for use in the prevention or treatment of Norovirus infection in a subject.
16. A method of increasing the stability of Norovirus genogroup I VLPs, comprising replacing the His residue in the sequence stretch Ala-Ala-Leu-Leu/Val-His-Tyr (SEQ ID NO: 57) in the P domain of a genogroup I VP1 capsid protein to Arg or Lys.

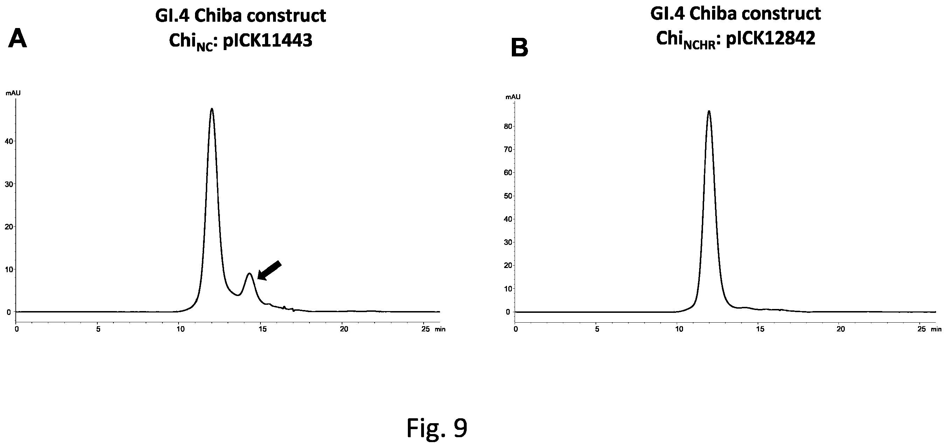

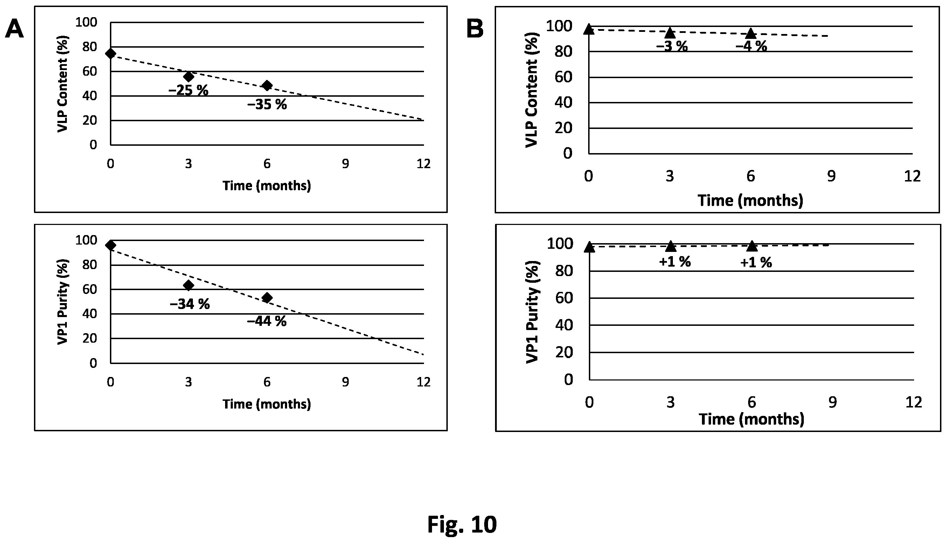

The inventors have found that norovirus (NoV) genogroup I VLPs generally have a stability lower than that of genogroup II NoV VLPs, which is problematic for providing stable VLP compositions and vaccines containing genogroup I VLPs. The inventors have further found a way to increase the stability of NoV genogroup I VLPs by modifying the amino acid sequence of genogroup I VP1 capsid protein. The modified VP1 capsid protein is the capsid protein or antigen of the invention. Thus, VLPs formed from the VP1 protein of the invention have improved stability. The improved stability is demonstrated in Example 3 and by the results reported in Figs. 9 and 10. Fig. 9 shows improved homogeneity and purity of the VLP of the invention by size-exclusion chromatography. Fig. 10 shows an improved stability trend and purity of the capsid protein of the invention (B) compared to the original capsid protein (A).

Moreover, if immunogenic compositions or vaccines are prepared that contain both genogroup I and genogroup II or IV VLPs, the stability of the genogroup I VLPs and the genogroup II or IV VLPs can be made more similar, whereby the compositions containing both types of VLPs are also more stable, which increases storage time and allows use of less harsh storage conditions (e.g. fridge temperature instead of freezing temperature) during storage and delivery of the vaccine of the invention.

### BRIEF DESCRIPTION OF THE DRAWINGS

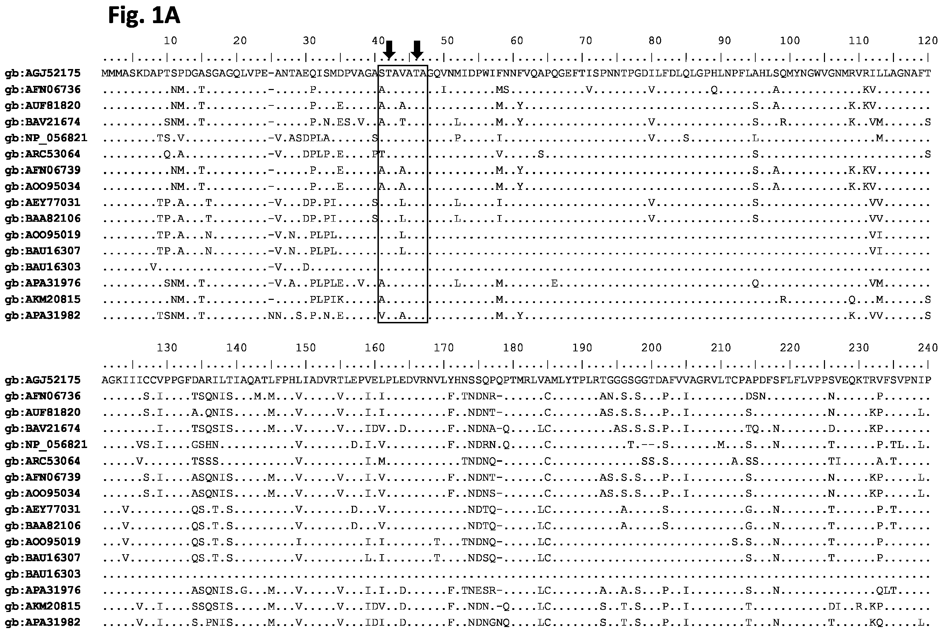

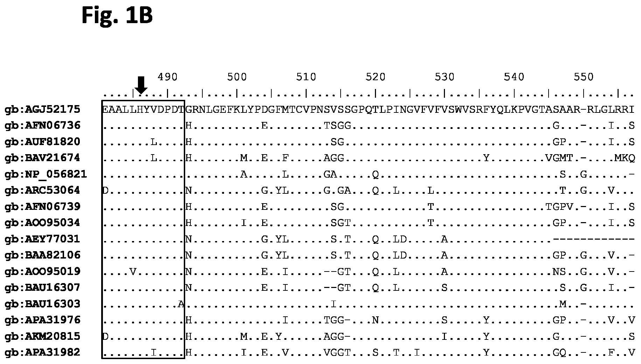

**Figure 1****:** Alignment of Norovirus capsid protein VP1 from different strains of genogroup I Noroviruses. The (S/A/T/V)TA(V/A/T/L)ATA motif and the conserved histidine residue of genogroup I sequences are indicated by a box. Fig. 1A shows a Norovirus GI alignment of the N-terminal part of VP1 proteins. Fig. 1B shows the Norovirus GI alignment of the C-terminal part of VP1 proteins. The sequences shown are partial sequences of complete VP1 amino acid sequences given in section "Amino Acid Sequences" below and in the electronic sequence listing.

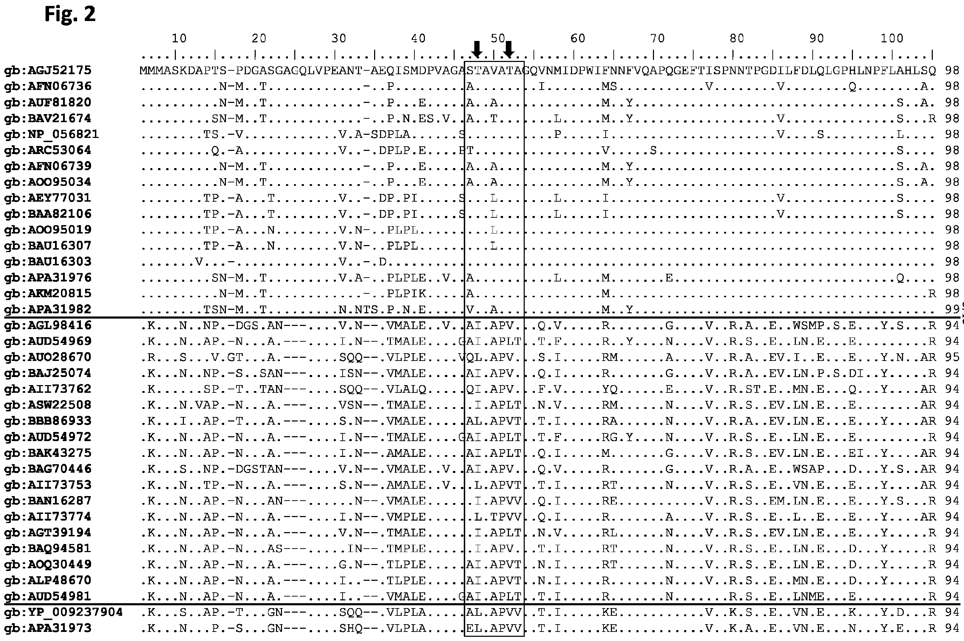

**Figure 2****:** Alignment of the N-terminal part of the Norovirus capsid protein VP1 from different strains of genogroup I, II and IV. Sequences of the different genogroups are separated by a horizontal line. The (S/A/T/V)TA(V/A/T/L)ATA motif of genogroup I and the corresponding region in the genogroup II and IV sequences is indicated by a box. The sequences shown are partial sequences of complete VP1 amino acid sequences given in section "Amino Acid Sequences" below and in the electronic sequence listing.

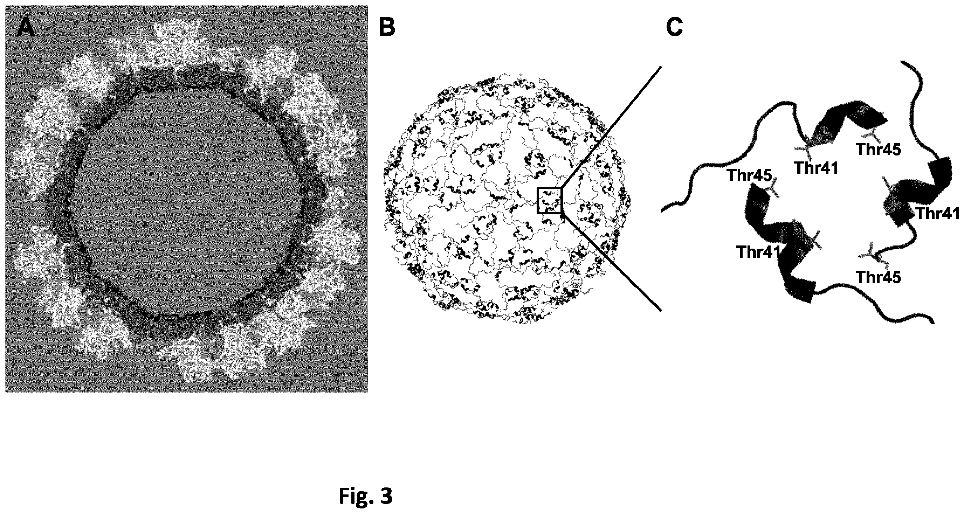

**Figure 3****:** Panel A shows a slab through the structure of a GI Norovirus-like particle (PDB:1IHM; white: protruding domain; grey: shell domain; black: inner surface). Panel B shows only the inner surface of the VLP (PDB: 1IHM amino acid residues 29-45). Panel C depicts a section of Panel B illustrating the configuration of the two conserved threonine residues of the genogroup I (S/A/T/V)TA(V/A/T/L)ATA motif at the inner surface of the VLP. Substitution of the two threonine residues with lysine and aspartic acid residues at the inner surface of the VLP can lead to formation of stabilizing ionic interactions or salt bridges.

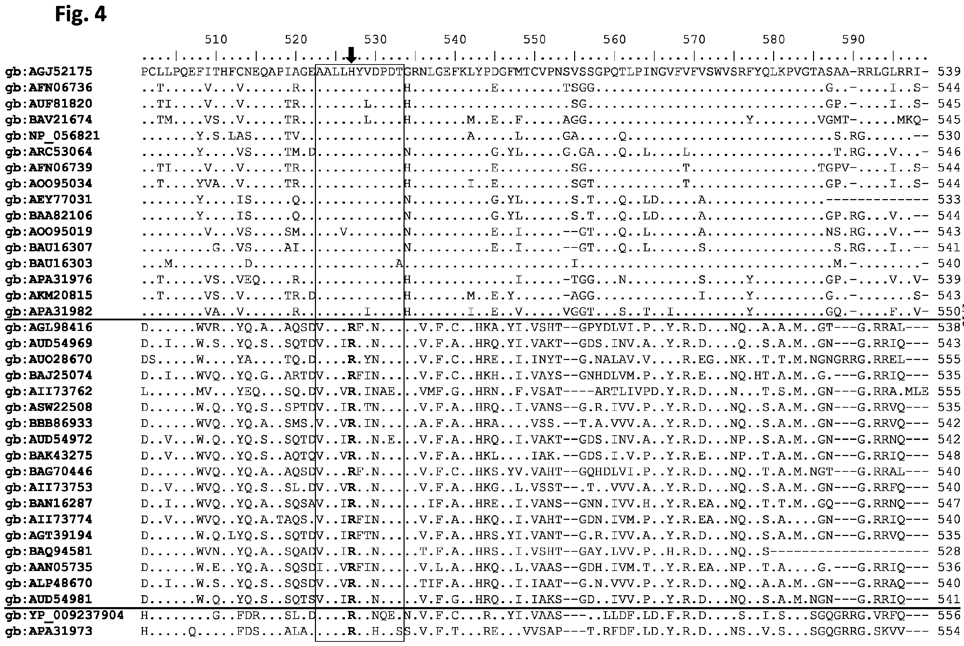

**Figure 4****:** Alignment of the C-terminal part of the Norovirus capsid protein VP1 from different strains of genogroup I, II and IV. Sequences of the different genogroups are separated by a horizontal line. The conserved histidine residue of genogroup I and the conserved arginine residue in genogroups II and IV sequences are indicated by a box. The sequences shown are partial sequences of complete VP1 amino acid sequences given in section "Amino Acid Sequences" below and in the electronic sequence listing.

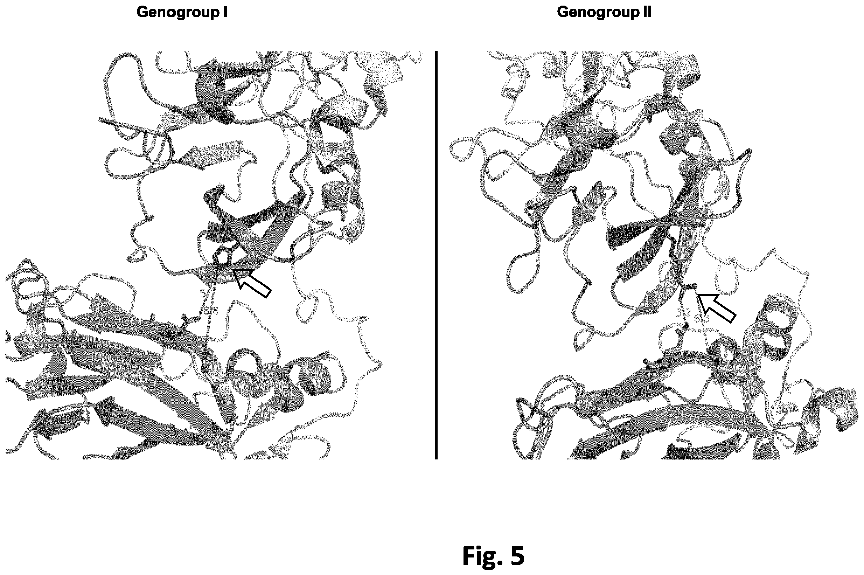

**Figure 5****:** Structure of a section of the protruding (upper part) and shell domain (lower part) of a genogroup I VP1 (PDB: 1IHM) and theoretical structure of the same region of a genogroup II VP1. The conserved histidine residue in genogroup I and the conserved arginine residue in genogroup II are indicated by an arrow. The arginine residue in genogroup II VP1 can participate in ionic interaction or formation of salt bridges between the protruding and shell domain and, thereby, be responsible for the better assembly and stability of genogroup II VP1-based virus-like particles.

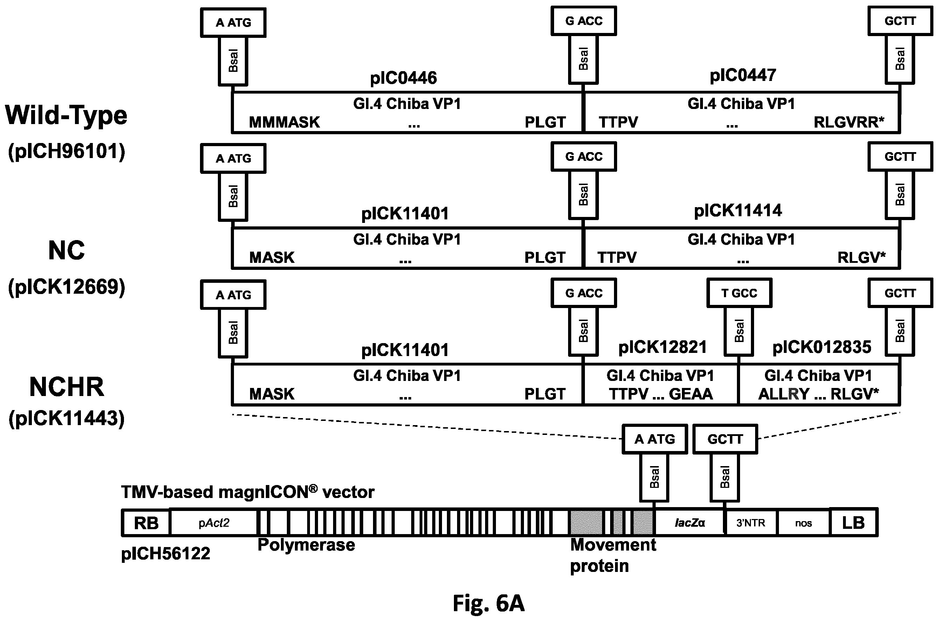

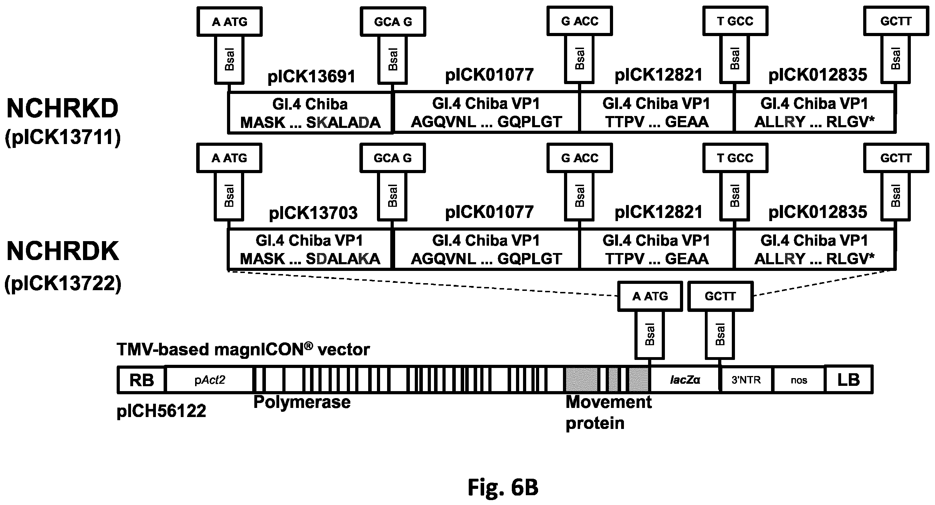

**Figure 6****:** Cloning schemes for Norovirus GI.4 Chiba VP1 wild-type, NC and NCHR (Fig. 6A) and NCHRKD and NCHRDK (Fig. 6B). Sequence modules for Norovirus GI.4 Chiba VP1 are cloned in a TMV-based viral binary expression vector using the Type IIS enzyme Bsal. Amino acid sequence ranges encoded by the modules are indicated. Overhangs flanking the modules after Bsal restriction digest are shown.

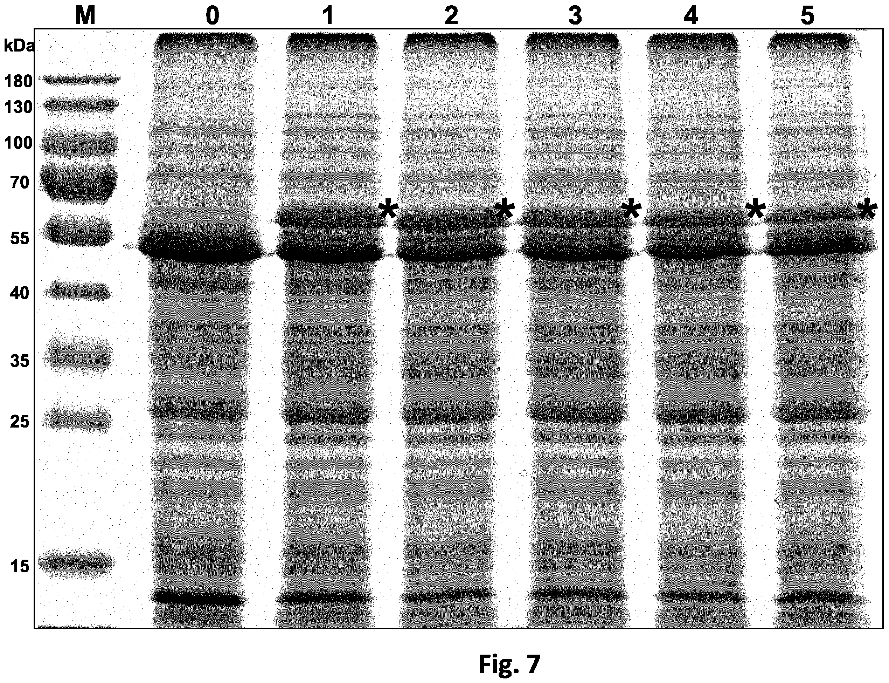

**Figure 7****:** Expression of wild-type and mutant versions of GI.4 Chiba 407 VP1 in *Nicotiana benthamiana* using magnICON^{®}. Laemmli total protein extracts were separated on a 12% polyacrylamide gel and Coomassie stained afterwards (M: size marker; 0: uninfiltrated plant tissue; 1: GI.4 Chiba 407 VP1 wild-type; 2: GI.4 Chiba 407 VP1 with single initiator methionine residue and deletion of the two C-terminal arginine residues; 3: GI.4 Chiba 407 VP1 with single initiator methionine residue, H472R and deletion of the two C-terminal arginine residues; 4: GI.4 Chiba 407 VP1 with single initiator methionine residue, T39K, T43D, H472R and deletion of the two C-terminal arginine residues; 5: GI.4 Chiba 407 VP1 with single initiator methionine residue, T39D, T43K, H472R and deletion of the two C-terminal arginine residues). The asterisk indicates the signal corresponding to GI.4 Chiba 407 VP1.

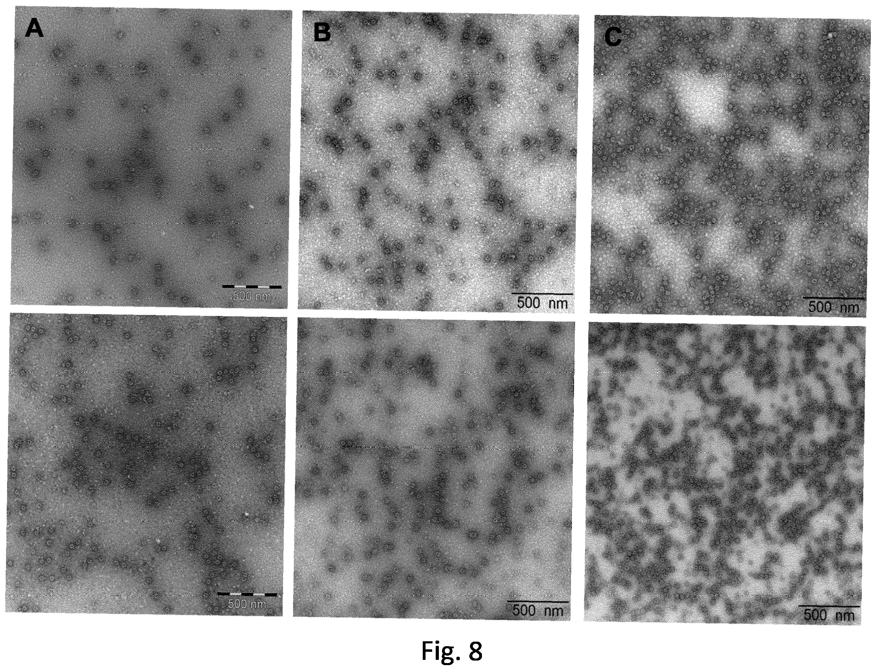

Figure 8: Transmission electron micrograph of two independent VLP batches each prepared using GI.4 Chiba 407 VP1 wild-type, NC or NCHR version. Transmission electron microscopy of UranyLess stained VLP samples prepared from GI.4 Chiba 407 VP1 A) wild-type, B) NC and C) NCHR version. The bar in the lower right of the images represents 500 nm.
**Figure 9****:** SE-HPLC of VLP derived from GI.4 Chiba 407 VP1 NC and NCHR version. Size-Exclusion-High-Performance Liquid Chromatography to analyze formation of VLP in samples prepared from GI.4 Chiba 407 VP1 A) NC and B) NCHR version. The black arrow indicates the second, smaller VLP species in the GI.4 Chiba 407 VP1 NC sample.
**Figure 10****:** Stability of GI.4 Chiba 407 NC and NCHR version. Stability trend of GI.4 Chiba 407 A) NC and B) NCHR version as indicated by VLP content in % (measured using SE-HPLC) and VP1 purity in % (measured using capillary gel electrophoresis).

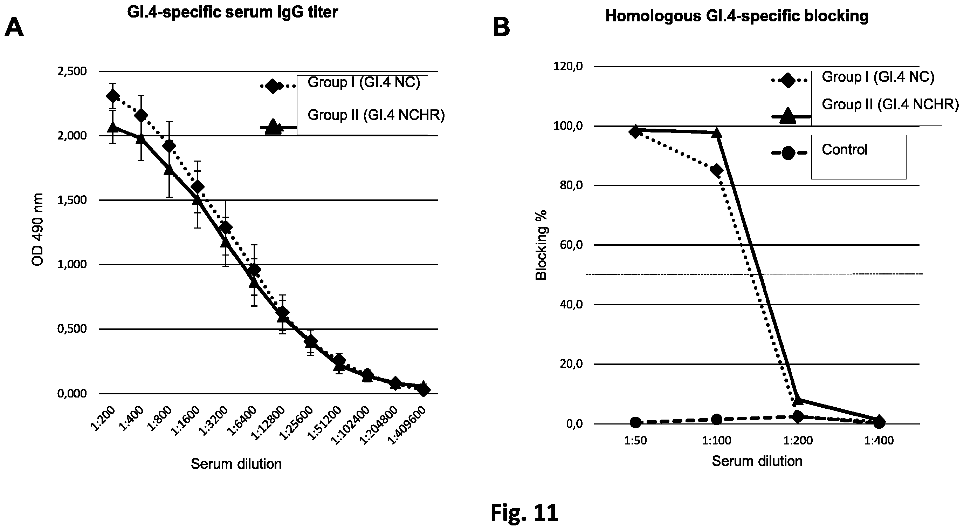

**Figure 11****:** Comparison of immunogenicity of GI.4 Chiba 407 NC and NCHR version. A) Homologous Norovirus VP1 antigen-specific serum IgG titration. Individual, serially diluted serum samples of immunized mice were analyzed for antibody levels at week 5 in ELISA. Shown are mean end-point titration curves of antibodies induced against GI.4 Chiba after VLP dose of 10 µg GI.4 Chiba NC or GI.4 Chiba NCHR. B) Group-wise pooled and two-fold titrated serum samples were tested for genotype-specific blocking (neutralizing) activity against homologous GI.4 Chiba variant VLPs using pig gastric mucin-based blocking assay. The blocking index (%) was calculated as 100% - [(OD wells with VLP and serum / OD wells without serum, "maximum binding") ×100%].

### DETAILED DESCRIPTION OF THE INVENTION

Noroviruses are non-enveloped single-stranded positive-sense RNA viruses. They belong to the family Calciviridae. The norovirus genome consists of three open reading frames (ORFs). ORF1 encodes the nonstructural proteins that are essential for virus replication, whereas ORF2 and ORF3 encode a major capsid protein VP1 and a minor structural protein VP2, respectively. VP1 self-assembles into virus-like particles (VLPs) that are morphologically and antigenically similar to native virions. Because NoVs are non-cultivatable in cell culture, much of the understanding of the biology of human NoVs has come from studies using VLPs.

There are five different genogroups of noroviruses (GI, GII, Gill, GIV, and GV) that can be further divided into genotypes. Genotypes are classified and referred to by Arabic numbers, frequently following the indication of the genogroup in Roman numbers. Examples of noroviruses are Norwalk virus (GenBank: AF093797.1), GI.1 strain Aichi/124-89/JP (GenBank: BAA834130), GI.2 strain Funabashi258/96/JP (GenBank: BAC05516), Maryland virus (MV, AY032605), GI.3 strain Shimizu/KK2866/JP (GenBank: AII73765), GI.4 strain Chiba407/87/JP (GenBank: BAA82106), GI.7 strain TCH-060/USA/2003 (GenBank: AEQ77282), GII.3 strain Kashiwa336/00/JP (GenBank: AAZ66774), GII.4 strain NL/2014/GII.4/Groningen01 (GeneBank: CRL46961), GII.4 strain Sydney/NSW0514/2012 /All (GenBank: AFV08795), GII.4 strain Aomori2/2006/JP (GenBank: BAG70446), GII.17 strain JP/2002/Saitama/T87 (GenBank: AII73747), Jena virus (JV, AJ01099), GII.17 strain JP/2013//Saitama5203 (GenBank: BAR63715), Seto virus (GenBank: AB031013), GII.17 strain C142/1978/GUF (GenBank: AGI17592), GIV.1 strain Ahrenshoop246/DEU/2012 (GenBank: AFN61315). There are many other norovirus strains the complete genomes of which are annotated in publicly available databases (www.viprbrc.org).

A key structural component of norovirus particles is the VP1 capsid protein. Another capsid protein is VP2 that is, however, not required for assembly of viral particles or VLPs. The size of the NoV particle varies between 23 and 40 nm in diameter. Depending on the size, the number of VP1 molecules per viral particle is generally either 60 or 180 molecules (http://viralzone.expasy.org/194).

The VP1 capsid protein can assemble, in the absence of the viral genetic material, to virus-like particles (VLPs) that resemble the viral particles in size and shape, but do not contain the NoV genome. VP1 alone (in the absence of VP2) is sufficient for forming VLPs. Thus, herein, VLPs comprise VP1 protein molecules and may further comprise VP2 protein molecules. In a preferred embodiment, the VLPs of the invention comprise VP1 capsid protein molecules but no VP2 protein molecules.

The NoV capsid protein VP1 has two domains, the shell (S) domain formed by, in the case of the Norwalk virus capsid protein (SEQ ID NO:10), amino acid residues 1-225 and the protruding (P) domain formed of residues 225-530 (Choi et al., PNAS 105 (2008) 9175-9180), see also Fig. 3. The S domain is involved in the formation of the icosahedral shell of the NoV capsid, whereas the P domain is involved in dimeric contacts of the VP1 protein. The dimers of the P domain project out from the icosahedral shell. The P domain is further subdivided into the P1 and P2 sub-domains. Amino acid residues 225-278 and 406-519 of the Norwalk virus VP1 capsid protein form the P1 sub-domain, and residues 279-405 form the distal P2 sub-domain (Choi *et al., ibid*.). In the case of the Chiba protein of SEQ ID NO: 3, residues 1-224 constitute the S domain, residues 224-540 constitute the P domain, residues 224-277 and 414-531 constitute the P1 domain, and residues 278-413 constitute P2 domain. The S, P, P1 and P2 domains of other genogroup I VP1 proteins such as those of SEQ ID NOs: 6 to 21 can be determined by sequence alignment to find the corresponding primary sequence segments that form the domains mentioned.

### Norovirus genogroup I VP1 capsid protein of the invention

The inventors have found variants of NoV genogroup I VP1 proteins that can assemble to VLPs of improved stability. The invention thus provides NoV genogroup I VP1 proteins. These VP1 proteins can assemble to, or can be assembled to, more stable VLPs. In one embodiment, the genogroup I VP1 protein of the invention has an Arg or Lys residue, preferably an Arg residue, at the position of a histidine residue that is highly conserved among genogroup I VP1 proteins. This position is position 474 in SEQ ID NO:1 or position 472 in SEQ ID NO:2 or 3. The corresponding position can be identified in other genogroup I VP1 proteins by alignment of an amino acid sequence of at least 500 amino acids to SEQ ID NO:3. Accordingly, the invention provides a NoV genogroup I VP1 capsid protein, wherein the amino acid sequence of said protein has at the position corresponding to His472 of SEQ ID NO:2, or at the position corresponding to Arg472 in SEQ ID NO: 3, 4 or 5, an Arg or Lys residue, preferably an Arg residue. Herein, the amino acid sequence shown in SEQ ID NO: 3 is the preferred reference sequence to identify the position in the VP1 sequence at which the capsid protein of the invention has an Arg or Lys residue.

The His amino acid corresponding to position 474 in SEQ ID NO:1 or position 472 in SEQ ID NO:2 is, in NoV genogroup I VP1 proteins, part of the sequence stretch Ala-Ala-Leu-Leu/Val-His- (SEQ ID NO: 61), preferably Ala-Ala-Leu-Leu/Val-His-Tyr (SEQ ID NO: 57). This sequence stretch is present in the P domain of the VP1 protein, preferably the P1 sub-domain of the VP1 protein. Thus, the NoV genogroup I VP1 capsid protein of the invention may be defined as follows. It has an amino acid sequence wherein the amino acid sequence stretch in the P domain, preferably the P1 domain, Ala-Ala-Leu-Leu/Val-His-Tyr (SEQ ID NO: 57) is modified to Ala-Ala-Leu-Leu/Val-Arg/Lys-Tyr (SEQ ID NO: 58), wherein Leu/Val means either Leu or Val, and Arg-/Lys means Arg or Lys. Preferably, the amino acid sequence stretch in the P domain Ala-Ala-Leu-Leu/Val-His-Tyr-Val/Leu/lle-Asp (SEQ ID NO: 59) is modified to Ala-Ala-Leu-Leu/Val-Arg/Lys-Tyr-Val/Leu/lle-Asp (SEQ ID NO: 60), wherein Val/Leu/Ile means either Val or Leu or Ile. The amino acid sequence of said protein preferably comprises at least 510, preferably at least 520, more preferably at least 530 amino acid residues.

The VP1 capsid protein of the invention is a NoV genogroup I protein. The classification of norovirus is based on the VP1 protein (Hansman et al., Journal of General Virology (2006), 87, 909-919). Thus, the established classification of NoV can be used for determining whether a given NoV VP1 capsid protein is a genogroup I capsid protein.

Embodiments of the VP1 capsid protein of the invention are as follows:
(i) the amino acid sequence of said protein consists of or comprises at least 500 contiguous amino acid residues of the amino acid sequence of any one of SEQ ID NOs: 6 to 21, except that the amino acid residue at the position corresponding to Arg472 in SEQ ID NO:3 is Arg or Lys; or
(ii) the amino acid sequence of said protein consists of or comprises at least 500 amino acid residues and has at least 80 %, preferably at least 85%, more preferably at least 90% and even more preferably at least 95% sequence identity over this length to a sequence segment of at least 500 contiguous amino acid residues of the amino acid sequence of any one of SEQ ID NOs: 6 to 21; or
(iii) the amino acid sequence of said protein has from 1 to 50, preferably from 1 to 40, more preferably from 1 to 30, and even more preferably from 1 to 20 deletions, substitutions, additions or insertions compared to a sequence segment of at least 500 contiguous amino acid residues of the amino acid sequence of any one of SEQ ID NOs: 6 to 21;
whereby in items (ii) or (iii) the amino acid residue at the position corresponding to Arg472 in SEQ ID NO:3 (or residue 469 of SEQ ID NO:6) is Arg or Lys, preferably Arg. In these embodiments, the minimum number of contiguous amino acid residues is preferably 510, more preferably 520, even more preferably 530, and most preferably 540 amino acid residues.

Other embodiments of the VP1 capsid protein of the invention are as follows:
(i') the amino acid sequence of said protein consists of or comprises the amino acid sequence from residue 43 to residue 540 of SEQ ID NO:3, 4 or 5; or
(ii') the amino acid sequence of said protein has at least 80 %, preferably at least 85%, more preferably at least 90%, and most preferably at least 95% sequence identity to the amino acid sequence from residue 43 to residue 540 of SEQ ID NO:3, 4 or 5; or
(iii') the amino acid sequence of said protein has from 1 to 50, preferably from 1 to 40, more preferably from 1 to 30, and even more preferably from 1 to 20 deletions, substitutions, additions or insertions compared to the amino acid sequence from residue 43 to residue 540 of SEQ ID NO:3, 4 or 5;
whereby in items (ii') or (iii') the amino acid at the position corresponding to Arg472 in SEQ ID NO:3, 4 or 5, respectively, is Arg or Lys, preferably Arg.

More preferred embodiments of the amino acid sequence of the VP1 capsid protein are as follows:
(i") the amino acid sequence of said protein consists of or comprises the amino acid sequence of SEQ ID NO:3, 4 or 5;
(ii") the amino acid sequence of said protein has at least 80 %, preferably at least 85%, more preferably at least 90%, and most preferably at least 95% sequence identity to the amino acid sequence of SEQ ID NO:3, 4 or 5;
(iii") the amino acid sequence of said protein has from 1 to 50, preferably from 1 to 40, more preferably from 1 to 30, and even more preferably from 1 to 20 deletions, substitutions, additions or insertions compared to the amino acid sequence of SEQ ID NO:3, 4 or 5;
whereby in items (ii") or (iii") the amino acid at the position corresponding to Arg472 in SEQ ID NO:3, 4 or 5, respectively, is Arg or Lys, preferably Arg.

In other embodiments, the capsid protein is defined by
(i‴) comprising a polypeptide molecule of an amino acid sequence comprising the amino acid sequence from residue 43 to residue 540 of SEQ ID NO:3, 4 or 5; or
(ii‴) comprising a polypeptide molecule of an amino acid sequence that has at least 80 %, preferably at least 85%, more preferably at least 90%, and most preferably at least 95% sequence identity to the amino acid sequence from residue 43 to residue 540 of SEQ ID NO:3, 4 or 5; or
(iii‴) comprising a polypeptide molecule of an amino acid sequence that has from 1 to 50, preferably from 1 to 40, more preferably from 1 to 30, and even more preferably from 1 to 20 deletions, substitutions, additions or insertions compared to the amino acid sequence from residue 43 to residue 540 of SEQ ID NO:3, 4 or 5;
whereby in items (ii‴) or (iii‴) the amino acid at the position corresponding to Arg472 in SEQ ID NO:3, 4 or 5, respectively, is Arg or Lys, preferably Arg.

A VP1 protein of genogroup I according to the invention, such as those defined above, may be of any one or more of the following genotypes: I.1, I.2, I.3, I.4, I.5, I.6, I.7, I.8, or I.9. Preferred are genotypes I.1 and I.4.

SEQ ID NOs: 1 to 21 are also referred to herein as "reference sequences". A "segment" or "portion" of an amino acid sequence is a partial sequence (or fragment) of contiguous amino acid residues of any given number of amino acid residues of the amino acid sequence which is referred to. The length of an amino acid sequence is measured by the number of amino acid residues it consists of including terminal residues. Amino acid sequence identities may be determined by protein sequence search and alignment programs freely accessible from internet, for example PROTEIN BLAST (https://blast.ncbi.nlm.nih.gov/Blast.cgi?PAGE=Proteins) and ExPASy (http://web.expasy.org/sim/). A comprehensive list of sequence alignment tools can be found at http://molbiol-tools.ca/Alignments.htm.

The NoV VP1 protein of the invention may be a VP1 protein of any NoV genogroup I genotpye, a fragment of such protein, a derivative of the protein or the fragment, or a protein comprising an amino acid sequence of a genogroup I NoV VP1 protein or derivative, as defined above.

The VP1 protein may have amino acid deletions, substitutions, additions or insertions compared to the reference sequences as indicated above. Among these, deletions, substitutions, and additions are preferred. The numbers of such alterations indicated above refer to the sum of all deletions, substitutions, additions and insertions. The term "insertion" relates to insertions within the amino acid sequence of a reference sequence, i.e. excluding additions at the C- or N-terminal end. The term "additions" means additions at the C- and/or N-terminal end of the amino acid sequence of a reference sequence. A deletion may be a deletion of a terminal or an internal amino acid residue of a reference sequence.

The VP1 protein of the invention may have the length of natural genogroup I VP1 proteins or may be a fragment thereof or derivative thereof. However, the VP1 protein of the invention should be capable of assembling to form VLPs. Herein, a VP1 protein of the invention has, as indicated above, preferably a minimum length of 500 amino acid residues and minimum identity or similarity to natural genogroup I VP1 proteins, as also indicated above. Whether VLPs are formed can be determined by established methods such as by electron microscopy (Laue M & Bannert N. , 2010, J Applied Microbiol., 109:1159-1168; Harris JR, 1999, Methods Mol Biol., 117:13-30; Pogan R et al., 2018, J Phys.: Condens. Matter, 30: 064006) or by size exclusion chromatography (Effio CL et al., 2016, Vaccine, 34:1259-1267).

The invention also provides a nucleic acid molecule encoding the capsid protein of the invention. The nucleic acid molecule may be a vector used for expressing the protein of the invention in host cells or a host organism. The nucleic acid molecule may contain a nucleic acid construct that contains a nucleotide sequence encoding the protein of the invention; the nucleic acid molecule may further contain genetic elements for expressing the nucleotide sequence. Further information on the nucleic acid molecule and the construct is given below in the section on the production of the protein of the invention.

### Virus-like particles (VLPs) of the invention

The invention also provides a VLP consisting of or comprising a VP1 capsid protein of the invention. A VLP is a particle consisting of virus structural protein(s), but does not contain viral nucleic acid. Preferably, a VLP is a particle consisting of virus structural protein(s), but does not contain noroviral nucleic acid encoding the VP1 or the VP1 and VP2 of said VLP. In the case of norovirus, a VLP consists of structural protein(s) VP1 or of VP1 and VP2 proteins (or any fragments or derivatives as described herein). The VLP of the invention comprises or consists of the VP1 capsid protein of the invention, such as those defined in any one of items (i) to (iii), items (i') to (iii'), items (i") to (iii"), or of items (i‴) to (iii‴) above. These proteins are preferably capable of forming VLPs.

The VLP of the invention preferably comprises at least 60, preferably at least 90, more preferably about 180 VP1 protein molecules. It is not necessary that all VP1 protein molecules of the VLP of the invention are genogroup I VP1 capsid protein molecules according to the invention. It is possible that the VLP also contains other VP1 protein molecules, preferably of genogroup I. However, in order to provide a high stabilizing effect of the VP1 protein of the invention on the VLP, at least 50 %, preferably at least 70%, more preferably at least 90% of the number of VP1 protein molecules of a VLP should be VP1 capsid protein molecules according to the invention. In a preferred embodiment, all VP1 protein molecules of a VLP are VP1 capsid protein molecules according to the invention. Thus, in one embodiment, the VLP of the invention comprises at least 60, preferably at least 90, more preferably about 180 VP1 capsid protein molecules according to the invention. The VLP may further comprise one or more VP2 protein molecules, preferably genogroup I VP2 protein molecules.

The VP1 capsid protein of the invention can assemble spontaneously into the VLP of the invention. Expression and production of the VP1 protein of the invention and production of the VLP is further explained below.

The invention also provides a method of increasing the stability of NoV genogroup I VLPs, comprising replacing the His residue in the sequence stretch Ala-Ala-Leu-Leu/Val-His-Tyr (SEQ ID NO: 57) in the P domain of a genogroup I VP1 capsid protein to Arg or Lys. The replacement is generally done by appropriate changes in a nucleic acid encoding a genogroup I VP1 protein, and expressing the modified VP1 encoding nucleic acid in a suitable expression host (further described below). The expressed VP1 protein is preferably assembled, in the presence or absence of further genogroup I VP1 protein of the same or a different genogroup I genotype, to the VLP of the invention.

### Immunogenic composition of the invention

The invention further provides an immunogenic composition comprising one or more NoV antigens, wherein at least one NoV antigen is the genogroup I VP1 capsid protein of the invention. Thus, the VP1 capsid protein of the invention is also referred to herein as the "antigen of the invention". The immunogenic composition is capable of generating an immune response in a mammal against the one or more NoV antigens in the composition. The immunogenic composition comprises as a NoV antigen, the VP1 capsid protein of the invention or, preferably, the VLP of the invention. The immunogenic composition may further comprise a pharmaceutically acceptable carrier and/or a vaccine adjuvant.

The immunogenic composition may comprise two or more different NoV antigens, such as two or more VP1 proteins, in order to generate an immune response in a subject against multiple NoV antigens at the same time, such as two, three or four different NoV antigens. The NoV antigen(s) used in the immunogenic composition of the invention depend on the NoV or NoVs against which immunization in a subject should be achieved using the vaccine of the invention. As noroviruses (NoVs) that cause infections in mammals evolve, the antigen(s) used in the immunogenic composition may be changed or adapted so as to cause immune response in a mammal against the NoVs considered a health risk at a given time or in a given season. The immunogenic composition of the invention contains at least the VP1 capsid or the VLP of the invention. Thus, it contains at least a NoV genogroup I antigen. The immunogenic composition may contain a second or further NoV genogroup antigen that may or may not be a genogroup I VP1 antigen of the invention; preferably a further NoV genogroup I antigen is also a genogroup I VP1 antigen of the invention. Thus, in one embodiment, the immunogenic composition comprises two or more genogroup I VP1 antigens, preferably of different genotypes, wherein the two or more genogroup I VP1 antigens are genogroup I VP1 antigens of the invention.

The immunogenic composition may further contain a NoV antigen from NoV genogroup Gil and/or GIV. Preferably, the immunogenic composition contains additionally a NoV antigen from NoV genogroup II (GII). The NoV antigens of genogroup GII and/or GIV are preferably VP1 capsid proteins.

The genogroup GII and/or GIV VP1 capsid protein that may be contained in the immunogenic composition may be as follows:
(a) the amino acid sequence of said protein consists of or comprises at least 500 contiguous amino acid residues of the amino acid sequence of any one of SEQ ID NOs: 22 to 53; or
(b) the amino acid sequence of said protein consists of or comprises at least 500 amino acid residues and has at least 80%, preferably at least 85%, more preferably at least 90% sequence identity over this length to a sequence segment of at least 500 contiguous amino acid residues of the amino acid sequence of any one of SEQ ID NOs: 22 to 53; or
(c) the amino acid sequence of said protein has from 1 to 50, preferably from 1 to 40, more preferably from 1 to 30 deletions, substitutions, additions or insertions compared to a sequence segment of at least 500 contiguous amino acid residues of the amino acid sequence of any one of SEQ ID NOs: 22 to 53.

Other embodiments of the VP1 capsid protein of the genogroup II or IV antigen are as follows:
(a') the amino acid sequence of said protein consists of or comprises the amino acid sequence of any one of SEQ ID NO: 22 to 53; or
(b') the amino acid sequence of said protein has at least 80 %, preferably at least 85%, more preferably at least 90%, and most preferably at least 95% sequence identity to the amino acid sequence of any one of SEQ ID NO: 22 to 53; or
(c') the amino acid sequence of said protein has from 1 to 50, preferably from 1 to 40, more preferably from 1 to 30, and even more preferably from 1 to 20 deletions, substitutions, additions or insertions compared to the amino acid sequence of any one of SEQ ID NO: 22 to 53.

The genotypes of these GII or GII capsid proteins are indicated in the section on the amino acid sequences.

Regarding the genotypes of the NoV antigens to be used in the immunogenic composition of the invention, there are no particular limitations. Preferred genotypes of genogroup I are any of the genogroup I genotypes given above, preferably genotypes 1.1 and I.4, and most preferably genotype I.4. An antigen of genogroup II may be an antigen of any one or more of the following genotypes: II.1, II.2, II.3, II.4, II.5, II.6, II.7, II.8, II.12, II.13, II.14, II.17, 11.21, II.22, II.24, or II.25, preferably II.4 and II.17, more preferably II.4. An antigen of genogroup IV may be an antigen of any one or more of the following genotypes: IV.1 or IV.3. Similarly as for the genogroup I antigens, the antigens of other genogroups are preferably VP1 proteins.

With regard to embodiments wherein an antigen from NoV genogroup I is combined with an antigen from NoV genogroup II, the following examples of immunogenic compositions may be mentioned:
one comprising an antigen of genotype I.1 and an antigen of genotype II.1;
one comprising an antigen of genotype I.1 and an antigen of genotype II.4;
one comprising an antigen of genotype I.1 and an antigen of genotype II.6;
one comprising an antigen of genotype I.4 and an antigen of genotype II.1;
one comprising an antigen of genotype I.4 and an antigen of genotype II.4;
one comprising an antigen of genotype I.4 and an antigen of genotype II.17;
one comprising an antigen of genotype I.4 and an antigen of genotype II.2;
one comprising an antigen of genotype I.1 and an antigen of genotype II.17;
one comprising an antigen of genotype I.1 and an antigen of genotype II.2; and
one comprising an antigen of genotype I.4 and an antigen of genotype II.6;
in all these embodiments of the immunogenic composition, the composition contains at least the VP1 capsid protein of the invention as a genogroup I antigen, preferably the genogroup I antigens are the VP1 capsid proteins of the invention. The antigens of genogroups I and II may be those defined above with reference to SEQ ID NOs. Preferred is an embodiment, wherein the immunogenic composition contains an antigen of genotype I.1 or I.4 (GI.4) and an antigen of genotype II.4 (GII.4) or GII.17 (GII.17). More preferred is an embodiment, wherein the immunogenic composition contains an antigen of genotype I.1 or I.4 and an antigen of genotype II.4. In all these embodiments, the immunogenic compositions may further contain a pharmaceutically acceptable carrier or a vaccine adjuvant as further explained below.

The immunogenic composition may, in a further embodiment, comprises two or more antigens from one NoV genogroup, preferably of genogroup II. The immunogenic composition may comprise, in addition to a genotype I VP1 capsid protein of the invention, two different antigens of genogroup II noroviruses, such as an antigen of a first genotype of a genogroup II norovirus and an antigen of a second genotype of a genogroup II norovirus. For example, the composition may comprise an antigen (e.g. VP1 protein) of a genotype II.1 NoV and an antigen (e.g. VP1 protein) of a genotype II.4 NoV. Alternatively, the composition may comprise an antigen (e.g. VP1 protein) of a genotype II.1 NoV and an antigen (e.g. VP1 protein) of a genotype II.17 NoV. Alternatively, the composition may comprise an antigen (e.g. VP1 protein) of a genotype II.4 NoV and an antigen (e.g. VP1 protein) of a genotype 11.17 NoV. These genogroup II antigens may be those defined above with reference to SEQ ID NOs.

Derivatives of an antigen as defined above are considered antigens of the genogroup and/or genotype to which the native antigen belongs. If an antigen such as a VP1 protein may, using this rule, belong to two different genogroups or genotypes, the antigen or derivative belongs to the genogroup or genotype to which the antigen or derivative is most similar in terms of amino acid sequence identity over the entire length of an amino acid sequence of a native VP1 protein such as of any one of SEQ ID NOs 1 to 56.

The immunogenic composition of the invention preferably contains the antigens in the form of NoV VLPs. Thus, the immunogenic composition of this embodiment may contain the VLP of the invention defined above. The immunogenic composition preferably contains the VLP of the invention and a NoV VLP of genogroup II and/or of genogroup IV antigens, preferably NoV VLP of genogroup II antigens. The VLP of genogroup II and/or of genogroup IV antigen are preferably VP1 proteins as mentioned above and of the genotypes mentioned above.

The immunogenic composition of the invention may contain a VLP comprising or consisting of a NoV antigen of genogroup I (that contains the VP1 capsid protein of the invention) and a VLP comprising or consisting of a NoV antigen of a second genogroup (e.g. genogroup II). The following immunogenic compositions may be mentioned as examples:
a composition comprising a VLP consisting of or comprising an antigen of genotype I.1 and a VLP consisting of or comprising an antigen of genotype II.1;
a composition comprising a VLP consisting of or comprising an antigen of genotype 1.1 and a VLP consisting of or comprising an antigen of genotype II.4;
a composition comprising a VLP consisting of or comprising an antigen of genotype 1.1 and a VLP consisting of or comprising an antigen of genotype II.6;
a composition comprising a VLP consisting of or comprising an antigen of genotype 1.1 and a VLP consisting of or comprising an antigen of genotype II.2;
a composition comprising a VLP consisting of or comprising an antigen of genotype 1.1 and a VLP consisting of or comprising an antigen of genotype 11.17;
a composition comprising a VLP consisting of or comprising an antigen of genotype I.4 and a VLP consisting of or comprising an antigen of genotype II.1;
a composition comprising a VLP consisting of or comprising an antigen of genotype I.4 and a VLP consisting of or comprising an antigen of genotype II.4; and
a composition comprising a VLP consisting of or comprising an antigen of genotype I.4 and a VLP consisting of or comprising an antigen of genotype II.6.
a composition comprising a VLP consisting of or comprising an antigen of genotype I.4 and a VLP consisting of or comprising an antigen of genotype II.2;
a composition comprising a VLP consisting of or comprising an antigen of genotype I.4 and a VLP consisting of or comprising an antigen of genotype II.17;
in all these embodiments, the VLP consisting of or comprising an antigen of genotype I is the VLP of the invention, wherein 100% of the VP1 protein molecules of said VLP may the VP1 capsid protein of the invention.

The immunogenic composition of the invention may comprise the genogroup I noroviral antigen (preferably VP1 protein) and the genogroup II noroviral antigen (also preferably VP1 protein) in a mass ratio range of from 1:1 to 1:6, preferably of from 1:1.5 to 1:5, more preferably of from 1:2 to 1:4. In other embodiments, the immunogenic composition of the invention comprises the genogroup I noroviral antigen and the genogroup II noroviral antigen in a mass ratio range of from 5:1 to 1:5, preferably of from 3:1 to 1:3, and more preferably of from 2:1 to 1:2, and even more preferably from 1.5:1 to 1:1.5.

In preferred embodiments, the immunogenic composition of the invention comprises the genogroup I VP1 capsid protein of the invention and a genogroup II noroviral antigen (preferably VP1 protein) in a mass ratio range of from 5:1 to 1:5, preferably of from 3:1 to 1:3, and more preferably of from 2:1 to 1:2, and even more preferably from 1.5:1 to 1:1.5.

In a further preferred embodiment, the immunogenic composition of the invention comprises the VLP of the invention and a NoV genogroup II VLP in a mass ratio range of from 5:1 to 1:5, preferably of from 3:1 to 1:3, and more preferably of from 2:1 to 1:2, and even more preferably from 1.5:1 to 1:1.5. The VLP of the invention is as defined above. The mass ratio may be measured e.g. by subjecting the VLPs to SDS-PAGE, staining and reading the stained intensities by a commercial reader. The measurement may be made before mixing the VLPs and subsequently mixing the desired VLPs in the desired ratio. Alternatively, VLP may be quantitated using mass spectroscopy such as MALDI-TOF.

The immunogenic composition may further contain a suitable carrier, in desired amounts and mixing ratios. For aqueous liquid compositions, a suitable carrier may be water or an aqueous solution that should be at an appropriate pH such as from 6 to 8, preferably from 6.7 to 7.5. The aqueous solution may contain, apart from water, a buffer, a tonicity agent and/or a preservative as required. For solid compositions, a liquid composition may be dried or lyophilized.

Examples of buffers are phosphate buffer, Tris buffer, acetate buffer, and citrate buffer. Examples of possible tonicity agents are sodium chloride, sucrose, glycerol, and trehalose. An example of a possible preservative is thimerosal. However, the immunogenic composition may be provided sterile without addition of a preservative.

The composition may be liquid or solid. If it is liquid, it may be a solution in water or an aqueous buffer. If it is solid, it may be a mixture containing the NoV genogroup I capsid protein, preferably containing the NoV VLPs of the invention. A preferred solid form is a lyophilized form.

The immunogenic composition is immunogenic in that it generates an immune response against the antigen(s) contained therein if the composition is administered to a subject, such as a human. In order to support the immune response in a subject, the composition may contain a vaccine adjuvant. An example of adjuvants is aluminum hydroxide (alum) that is a generally know adjuvant for vaccines or other aluminum salts. In one embodiment, however, the immunogenic composition (and the vaccine) does not contain an adjuvant. In a preferred embodiment, the composition (and the vaccine) does not contain alum or another aluminum salt as adjuvant.

For preparing the immunogenic composition of the invention, the one or more antigens, preferably in the form of VLPs, may be mixed preferably in or with a suitable carrier or medium. The carrier or medium may be water or an aqueous medium such as a solution. The aqueous medium may contain a buffer to control the pH and may contain physiologic saline and/or other additives. Possible additives can be, but are not limited to, sucrose, glycerol, trehalose. The NoV antigens may be stored in an aqueous medium until the immunogenic composition or the vaccine of the invention are produced. For longer storage times, it may be frozen or lyophilized. After production of the immunogenic composition, it may be sterilized, e.g. by sterile filtration and stored. Storage may be in liquid form or frozen. It may also be stored after lyophilization as a dry powder.

The lyophilization of immunogenic composition and vaccines is well known in the art. Typically the composition is freeze dried in the presence of agents to protect the antigen and/or adjuvant of the invention during the lyophilization process and to yield powders with desirable characteristics. Sugars such as sucrose, mannitol, trehalose, or lactose (present at an initial concentration of 10-200 mg/mL) are commonly used for cryoprotection and lyoprotection of protein antigens and to yield lyophilized cake or powders with desirable characteristics. Lyophilized compositions may be more stable. Other drying technologies, such as spray drying or spray freeze drying may also be used.

### Vaccine of the invention

The vaccine of the invention comprises the immunogenic composition of the invention in a form suitable for administration to a subject or in a form that can, prior to administration, be easily brought in a form suitable for administration to a subject. Preferably, the vaccine contains the immunogenic composition in a suitable dosage form, preferably as a single or unit dose dosage form containing the amount of antigen to be administered to a subject at a single administration.

The form suitable for administration is related to the mode of administration. For example, the vaccine may be a solid formulation that can be reconstituted prior to administration by addition of a predetermined amount of water or aqueous solution, suspension or emulsion. In this way, solutions for administration e.g. by injection can be made shortly before administration. Alternatively, the vaccine may be a solid formulation for intranasal administration to a subject. Further, the vaccine may be a liquid preparation, preferably an aqueous liquid formulation, for administration by injection. In the latter case, the vaccine may be an aqueous solution as a unit dosage form.

The vaccine may comprise, apart from the immunogenic composition, one or more pharmaceutical excipients or carriers. Excipients may be liquid or solid. Liquid excipients include, without limitation, water, alcohol, saline, and buffered solutions. Other possible excipients include, without limitation, preservatives and other additives such as antimicrobials, anti-oxidants, chelating agents, buffer substances. The immunogenic composition of the invention may itself be a vaccine in the sense of the invention.

The vaccine is an anti-NoV vaccine. It is at the same time a pharmaceutical composition. The vaccine is generally used for preventing or treating NoV infection in a subject, or for suppressing the severity of a NoV infection. For preventing or treating norovirus infection, or for suppressing the severity of a NoV infection, the immunogenic composition or vaccine of the invention may be administered to a subject. Subjects in which NoV may be prevented or treated or in which the severity of a NoV infection may be suppressed are mammals, preferably humans. Among humans, both children and adults may be subjects for preventing or treating NoV infection. Among humans, children are preferred for achieving immunization early in life. Human subjects are considered children up to the age of 16. Preferably, the NoV vaccine is used in children of age between 1 and 16, preferably 2 to 14, and more preferably from 3 to 12 years of age.

Regarding the route of administration of the vaccine, the invention is not limited. The vaccine of the invention is preferably administered to a subject parenterally, e.g. by injection. For administration by injection, the vaccine may be formulated as a liquid or as a solid for reconstitution to form a liquid. The parenteral administration by injection may be intravenous, intradermal, intramuscular or subcutaneous administration. Preferred are intradermal, intramuscular or subcutaneous administration, more preferably intradermal and intramuscular administration. In one embodiment, the vaccine is administered intradermally. In another embodiment, the vaccine is administered intramuscularly. The vaccine may also be administered intranasally. In this case, the composition or vaccine may a liquid, preferably an aqueous solution. Alternatively, the composition or vaccine for intranasal administration may in solid form, such as in a lyophilized form.

When the vaccine is administered to a human subject, the NoV antigen(s) may be administered in an amount of from 10 to 1000 µg, preferably from 30 to 300 µg, more preferably from 55 to 150 µg of NoV antigen. If the vaccine contains more than one NoV antigen, these amounts relate to the sum of the amounts of the individual NoV antigens. If the vaccine contains antigens of genogroup I and antigens of genogroup II, the amount of the genogroup II antigen(s) may be the same or higher than the genogroup I antigen(s). The amount of the genogroup II antigen(s) may be from 1.5 to 6 times, preferably from 2.0 to 5 times, more preferably from 2.5 to 4.5 times, and even more preferably from 3.0 to 4.0 times the amount of the genogroup I antigen(s) in terms of mass.

The vaccine may be packaged in a single-dose or multiple dose form in a container that contains the desired amount of the vaccine suitable for administration. Preferred are single-dose forms, where the single dose contains the administration amount of NoV antigen as given above. The single-dose form may comprise from 10 to 1000 µg, preferably from 30 to 300 µg, more preferably from 55 to 150 µg of NoV antigen(s). If the vaccine contains antigens of genogroup I and antigens of genogroup II, the ratios of these antigens may be as defined in the previous paragraph.

The vaccine may be administered once or twice to a subject for improving the immunity against NoV infection. If the vaccine is be administered twice, the second administration may be made within 2 to 8 weeks, preferably within 3 to 5 weeks after the first administration of the vaccine.

The vaccine of the invention may also contain antigens against other infectious diseases for generating immune protection not only against NoV, but also against other viruses. The vaccine may, for example, comprise rotavirus antigens to generate protection against NoV and rotavirus.

A method of producing an immunogenic composition or vaccine against NoV infection may comprise expressing the VP1 capsid protein of the invention in an expression host, assembling the expressed VP1 capsid protein to form VLPs, and formulating the VLPs for administration to a subject.

### Production of NoV antigens

NoV antigens as described above can be expressed in and purified from different production hosts including mammalian cells, insect cells and plants or plant cells (for review: Herbst-Kralovetz, M., Mason, H. S. & Chen, Q. 2010, Exp. Rev. Vaccines, 9:299-307). Reliable NoV antigen and NoV VLP purification protocols and modifications thereof have been described for insect cells using baculoviral expression system (Jiang, X. et al., 1992, J.Virol., 66:6527-6532; Prasad BVV, Hardy D, Estes M. 2000, J. Infect. Dis., 181:S317-S321; Huhti, L., et al., 2010, Arch. Virol., 155:1855-1858; Koho, T., et al., 2012, J. Virol. Methods, 181:6-11; Huhti, L., et al., 2013, Arch. Virol., 158:933-942; WO2013192604) and for plants (Santi L. et al., 2008, Vaccine, 26:1846-1854; Lai, H. & Chen, Q. 2012, Plant Cell Rep., 31:573-584). EP2601970 may also be considered regarding the production of NoV VLPs.

Although there is no difference observed in the structure and immunogenic properties of VLPs isolated from insect and plant cells, a preferred system for VLP production is plant-based, as this allows avoiding baculoviral impurities in the antigen or VLPs isolated from plant tissues. In addition, plant-based transient expression systems, unlike the baculoviral one, are easily scalable. NoV antigen genes can be successfully expressed in plants using plant virus-based expression system called magnICON^{®} (Gleba et al., 2005, Vaccine, 23:17-18; Marillonnet et al., 2005, Nat. Biotechnol., 23:718-723; Gleba et al., Curr. Opin. Biotechnol., 2007, 18:134-141; Klimyuk, V., et al., 2014, Curr. Top. Microbiol. Immunol., 375:127-154) that allows to easily express viral VLPs in *Nicotiana benthamiana* plants (Zahin, M. et al., 2016, PLoS One, 11(8):e0160995).

In more detail, the capsid protein of the invention and other antigens to be included in the immunogenic composition or vaccine may be produced by known methods of protein expression in a standard expression system. For producing the capsid protein or antigen, a nucleotide sequence encoding it may be expressed in a suitable host organism. Methods usable for producing and purifying a protein of interest have been described in the prior art and any such methods may be used. If a eukaryotic expression system is used, one or more introns may be inserted in the coding sequence of the antigen.

Particularly efficient expression methods are plant expression systems that are also known in the prior art. A possible way of achieving expression of a nucleotide sequence of interest encoding an antigen according to the invention in plants is the use of self-replicating (viral) replicons containing the nucleotide sequence encoding the antigen. Plant viral expression systems have been described in many publications, such as in WO2012019660, WO2008028661, WO2006003018, WO2005071090, WO2005049839, WO2006012906, WO02101006, WO2007137788 or WO02068664 and many more publications are cited in these documents. Various methods for introducing a nucleic acid molecule, such as a DNA molecule, into a plant or plant part for transient expression are known. Agrobacteria may be used for transfecting plants with the nucleic acid molecule (vector) or nucleic acid construct e.g. by agroinfiltration or spraying with agrobacterial suspensions. For references, see WO 2012019660, WO 2014187571, or WO 2013149726.

In embodiments wherein strong expression of the antigen is desired, a nucleic acid construct containing a nucleotide sequence encoding the capsid protein may encode a viral vector that can replicate in plant cells to form replicons of the viral vector. In order to be replicating, the viral vector and the replicons may contain an origin of replication that can be recognized by a nucleic acid polymerase present in plant cells, such as by the viral polymerase expressed from the replicon. In case of RNA viral vectors (referred to as "RNA replicons"), the replicons may be formed by transcription under the control of a promoter active in plant cells, from the DNA construct after the latter has been introduced into plant cell nuclei. In case of DNA replicons, the replicons may be formed by recombination between two recombination sites flanking the sequence encoding the viral replicon in the DNA construct, e.g. as described in WO00/17365 and WO 99/22003. If the replicon is encoded by the DNA construct, RNA replicons are preferred. Use of DNA and RNA viral vectors (DNA or RNA replicons) has been extensively described in the literature over the years. Some examples are the following patent publications: WO2008028661, WO2007137788, WO 2006003018, WO2005071090, WO2005049839, WO02097080, WO02088369, WO02068664. Examples of DNA viral vectors are those based on geminiviruses. For the present invention, viral vectors or replicons based on plant RNA viruses, notably those based on plus-sense single-stranded RNA viruses may be preferably used. Accordingly, the viral replicon may be a plus-sense single-stranded RNA replicon. Examples of such viral vectors are those based on tobacco mosaic virus (TMV) and potexvirus X (PVX). "Based on" means that the viral vector uses the replication system such as the replicase and/or other proteins involved in replication of these viruses. Potexvirus-based viral vectors and expression systems are described in EP2061890 or WO2008/028661.

The capsid protein may be expressed in a multi-cellular plant or a part thereof, notably a higher plant or parts thereof. Both monocot and dicot (crop) plants can be used. Common plants usable for expressing proteins include *Nicotiana tabacum* and *Nicotiana benthamiana.* However, many others can be employed as well.

Generally, the capsid protein may be expressed in the cytosol of cells of the plants or plant parts. In this case, no signal peptide directing the protein of interest into a particular compartment is added to the enzyme. Alternatively, the capsid protein or antigen can be expressed in or targeted into chloroplasts of the plants or be secreted into the extracellular space; in these cases, an N-terminal pre-sequence, such as a plastid transit peptide or a signal sequence for targeting to the extracellular space, is added to the N-terminal or C-terminal end, preferably the C-terminal end, of the capsid protein as the protein of interest.

In the next step, plant material containing expressed capsid protein from a plant having expressed the antigen is harvested. Plant material may e.g. be leaves, roots, tubers, or seeds, or a crushed, milled or comminuted product of leaves, roots, tubers, or seeds. The capsid protein may then be extracted from the plant material using an aqueous buffer. This may include that the plant material is homogenized and insoluble material may be removed by centrifugation or filtration. Soluble components including the capsid protein will be extracted into the aqueous buffer to produce an capsid protein solution in the aqueous buffer. The aqueous buffer may contain an inorganic or organic acid or salts thereof and may have a pH as defined below for the aqueous solution as a composition of the invention. Further, the aqueous buffer may contain salt and/or a sulfhydryl compound. The capsid protein in the obtained antigen preparation may then be further purified using standard methods of protein purification, such as chromatographic methods.

The capsid protein, being a NoV VP1 protein or derivative, can form VLPs. These VLPs generally form spontaneously in the aqueous buffer used in extraction and purification. Formation of VLPs can be verified e.g. using electron microscopy.

If the immunogenic composition or vaccine of the invention contains two or more NoV antigens, the two or more antigens may be expressed and purified separately and then mixed in desired ratios when preparing the composition or vaccine. It is also possible to co-express two or more capsid protein or antigens in the same plant cells or plants and purifying the together as a mixture.

Construct engineering for antigen expression including VP1 antigens, expression of antigens and purification are described in Example 1 and 2. Figure 6 shows generation of expression vector containing GI.4 antigen coding sequence. Similar approach using type IIS restriction enzymes can be used for cloning any sequence of interest into the expression vector. Figures 7 shows wild type and mutant GI.4 VP1 protein expression on . Coomassie-stained gel.

### EXAMPLES

### EXAMPLE 1

### Construction and functional testing of expression vectors for Norovirus VP1 wild-type and mutant versions

The sequence corresponding to the VP1 capsid protein of Norovirus GI.4 Chiba 407 (Japan, 1987) (GenBank accession no.: BAB18267, 544 amino acid residues, SEQ ID No: 1, Fig. 1) was generated using gene synthesis and flanking Type IIS restriction enzyme Bsal sites were added for cloning. The Norovirus GI.4 Chiba 407 modules were cloned in TMV-based viral expression vectors (magnICON^{®} system, see below) using the Type IIS restriction enzyme Bsal which generates different, customized overhangs for each module and thereby allowing assembly of the final construct by removing these restriction sites (Engler, C., Kandzia, R. & Marillonnet, S. 2008, PLoS One, 3:e3647).

Analysis of GI.4 Chiba 407 VP1 protein revealed that the wild-type protein sequence was lacking two N-terminal methionine residues and the two C-terminal arginine residues resulting in a difference of the theoretical and determined protein mass. Therefore, an N- and C-terminal truncated NC variant (SEQ ID No: 2) lacking two N-terminal methionine and two C-terminal arginine residues of GI.4 Chiba 407 VP1 was generated to provide a matching variant for theoretic and determined mass. The respective Norovirus GI.4 Chiba 407 VP1 modules were generated via PCR with flanking Type IIS restriction enzyme Bsal sites and cloned in TMV-based magnICON^{®} viral expression vectors using these Bsal sites. The NC version of SEQ ID No: 2 (compared to wild-type sequence: N-terminally ΔMM, C-terminally ΔRR) was used as basis for the introduction of further mutations.

GI.4 VLPs, in comparison with GII.4 VLPs, showed lower homogeneity and also lower stability. Furthermore, for GI.4 Chiba 407 wild-type and NC version VP1, an antigen-related high molecular weight product was co-purified and co-formulated with the VLPs in a reproducible manner. Since this behavior was not observed for GII.4 strains, a comparative sequence and structure analysis was conducted. A single arginine amino acid residue within a conserved but structurally flexible region of the P1 domain of genogroups II and IV strains was identified, which can lead to formation of salt bridges between shell and protruding domain, thereby improving conformation, multimerization and assembly of VP1 and stabilizing the resulting VLPs. In genogroup I strains, the amino acid residue at this position is histidine. Therefore, this histidine residue in the VP1 sequence of GI.4 Chiba 407 was replaced with arginine (H474R in wild-type and H472R for NC variant). The respective Norovirus GI.4 Chiba 407 VP1 NC version modules were generated via PCR with flanking Type IIS restriction enzyme Bsal sites and cloned in TMV-based magnICON^{®} viral expression vectors using these Bsal sites resulting in the NCHR version (SEQ ID No: 3) of GI.4 Chiba 407 VP1 (compared to wild-type sequence: N-terminally ΔMM, H474R, C-terminally ΔRR).

To further improve GI.4 Chiba 407 VLP stability and integrity, the inner shell domain was modified by substitution of residues to allow additional formation of shell-stabilizing salt bridges at the inner surface of the VLPs, i.e., the N-terminal STALATA motif was modified to SKALADA or SDALAKA. The respective Norovirus GI.4 Chiba 407 VP1 NCHR version modules were generated via PCR with flanking Type IIS restriction enzyme Bsal sites and cloned in TMV-based magnICON^{®} viral expression vectors using these Bsal sites resulting in the NCHRKD (SEQ ID No: 4) and NCHRDK (SEQ ID No: 5) version of GI.4 Chiba 407 VP1 (compared to wild-type sequence: N-terminally ΔMM, T41K/D, T45D/K, H474R, C-terminally ΔRR).

Viral binary expression vectors were developed based on the magnICON^{®} technology (Gleba, Y., Klimyuk, V. & Marillonnet, S., 2005, Vaccine, 2005, 23:2042-2048; Gleba, Y., Klimyuk, V. & Marillonnet, S. 2007, Curr. Opin. Biotechnol., 18:134-141) using elements from Tobamo viruses (Tobacco Mosaic Virus, TMV), i.e. from the cDNAs of two closely related plant viruses, TVCV (turnip vein clearing virus; Lartey, R.T., Lane, L.C. & Melcher, U. 1994, Arch. Virol., 138:287-298; Lartey, R.T., Voss, T.C. & Melcher, U. 1995, Gene, 166:331-332) and crTMV (crucifer-infecting tobamovirus; Dorokhov, Y.L., Ivanov, P.A., Novikov, V.K., et al., 1994, FEBS Lett., 350:5-8). The resulting vectors are called 'TMV-based', since both parental viruses are tobamoviruses and related to the well-known tobacco mosaic virus (TMV). All three viruses (TVCV, crTMV and TMV) are positive-strand RNA viruses and have the same overall structure and mode of replication. Basically, the viruses encode an RNA-dependent RNA polymerase (RdRP), the Movement Protein (MP) and the Coat Protein (CP). The RdRP replicates the full viral RNA transcript (genomic RNA) as well as the two subgenomic RNAs (sgRNAs) that are required for expression of the two other viral proteins, MP and CP. The MP is required for short distance cell-to-cell movement of the viral genomic RNA within the infiltrated leaf. The CP is required for formation of viral particles and long distance systemic movement from leaf to leaf via the vascular system. Formation of viral particles is not required for cell-to-cell movement. Therefore, the CP was eliminated from the viral vectors and replaced with the gene of interest. Thus, the viral vector is unable to produce viral particles and the gene of interest is expressed at higher levels. In addition to the viral protein genes and the gene of interest, the viral vector also contains the 5' and 3' non-translated (5' ntr and 3' ntr) viral sequences which are essential for replication (Marillonnet, S., Giritch, A., Gils, M. et al., 2004, Proc. Natl. Acad. Sci. U S A., 101:6852-7).

For efficient expression of the TMV-based viral vector in plant cells, the cDNA of the viral vector was cloned between a plant promoter and a plant terminator (Act2 and nos) (Marillonnet, S., Giritch, A., Gils, M. et al., 2004, Proc. Natl. Acad. Sci. U S A., 101:6852-6857) and plant introns were added within the RdRP and MP sequences (Marillonnet, S., Thoeringer, C., Kandzia, R. et al., 2005, Nat. Biotechnol., 23:718-723). For efficient delivery of TMV-based viral vectors to plant cells, we used *Agrobacterium tumefaciens.* Therefore, the complete viral vector (plant promoter, TMV-based viral vector sequences with gene of interest, plant terminator) was cloned between the T-DNA left and right borders of a binary vector. The elements of the binary vector are a pVS1 origin (Hajdukiewicz, P., Svab, Z. & Maliga, P., 1994, Plant Mol Biol., 25:989-994) for plasmid replication in Agrobacterium, a co/E1 origin for plasmid replication in *E. coli,* a *nptIII* kanamycin antibiotic resistance gene (Frisch, D.A., Harris-Haller, L.W., Yokubaitis, N.T. et al., 1995, Plant Mol. Biol., 27:405-409) and T-DNA left and right borders (Frisch, D.A., Harris-Haller, L.W., Yokubaitis, N.T. et al., 1995, Plant Mol. Biol., 27:405-409) to delimitate the ends the DNA transferred to plant cells. To facilitate blue/white selection a *lacZ*α cassette amplified from pUC19 was inserted between two Bsal restriction sites which allow seamless in frame cloning of the gene of interest. Therefore, during initial construction of the viral vectors, all naturally occurring Bsal recognition sites were removed to allow easy and robust cloning of the gene of interest.

For functional testing of expression of the recombinant proteins, the selected *Agrobacterium* strain harboring the TMV-based expression vector was grown in liquid LBS medium with soya peptone (Duchefa Biochemie, Haarlem, The Netherlands) replacing tryptone, and supplemented with 50 mg/mL rifampicin and 50 mg/mL kanamycin. Agrobacterial cultures were grown at 28°C until OD600 reaches 2 to 4. Infiltration solution is prepared by diluting the agrobacterial culture in infiltration buffer (10 mM MES, pH 5.5, 10 mM MgSO₄) to a defined cell concentration (equivalent to a 1000-fold dilution of a culture with OD₆₀₀ of 2.0).

Leaves of *Nicotiana benthamiana* plants grown under controlled and standardized conditions for 6-8 weeks were infiltrated with the agrobacterial infiltration solution using a needle-less syringe and then kept in the greenhouse for approximately 7 days for expression and accumulation of the recombinant protein. Plant leaf material was then harvested, ground in liquid nitrogen to a fine powder and proteins extracted using Laemmli buffer. The protein extracts were analyzed by SDS-polyacrylamid-gel electrophoresis and proteins visualized by Coomassie staining.

### Example 2

### Norovirus VLPs purification from plant material

Norovirus VP1 VLPs were purified as described below. The five weeks old *Nicotiana benthamiana* plants were vacuum-infiltrated (80-100 mbar for 3-4 minutes) with diluted *Agrobacterium tumefaciens* cultures carrying TMV-based assembled magnICON^{®} vectors (Gleba et al., 2005, Vaccine, 23:17-18; Marillonnet et al., 2005, Nat. Biotechnol., 23:718-723; Gleba et al., Curr. Opin. Biotechnol., 2007, 18:134-141; Klimyuk, V., et al., 2014, Curr. Top. Microbiol. Immunol., 375:127-154) that allows to easily express viral VLPs in *Nicotiana benthamiana* plants (Zahin, M. et al., 2016, PLoS One, 11(8):e0160995). Plant material was harvested 6-14 days post infiltration. A harvesting time point 7-8 days post infiltration results in the highest expression level.

The green biomass was homogenized in the presence of two volumes neutral buffer (i.e. 15 g biomass and 30 mL 100 mM Tris, 5 mM Na₂S₂O₅ pH 7.5). For clarification, the plant homogenate was centrifuged 20 min. at 15.000 × g. The resulting extract was further clarified by filtration using a Millipore^{®} glass fiber filter (AP25).

High molecular weight components were sedimented by ultracentrifugation (150.000xg for 90 min.). The pellet was suspended in 1 mL of 20 mM histidine, 137 mM NaCl pH 6.0 and clarified by 15.000 × g centrifugation for 20 minutes. The VLP containing supernatant was placed on the top of a 30% sucrose cushion (in 20 mM histidine, 137 mM NaCl pH 6.5). Ultracentrifugation was performed for 90 min. at 150.000 × g. The resulting pellet was resuspended in 20 mM histidine, 137 mM NaCl pH 6.5.

### EXAMPLE 3

### Characterization of purified VP1 VLPs

### Transmission electron microscopy (TEM)

The presence of assembled VLPs was monitored by transmission electron microscopy. TEM micrographs were taken to visualize the size, dispersity, and icosahedral morphology of purified VLPs.

Samples were prepared by using the single droplet negative staining technique (J. Robbin Harris, Methods in Molecular Biology 117: Electron Microscopy Methods and Protocols chapter S. 13 - 30). Pre-coated grids (Formvar-Carbon, Cu 200 mesh, FCF200-Cu-25, Science Services GmbH, Munich, Germany) as support films and UranyLess (E22409, Science Services GmbH, Munich, Germany) as contrast solution were used. 10 µl of each sample was applied to a grid and incubated for 10 min. The grid surface was washed by touching the coated side on a drop of ultrapure water for 5 sec. Excess water was removed by filter paper. VLPs were stained twice with ready-to-use UranyLess for 1 min each time. After drying overnight the grids were observed with an EM 900 transmission electron microscope (Carl Zeiss Microscopy, Oberkochen, Germany) operating at 80kV. Micrographs were taken with a Variospeed SSCCD camera SM-1k-120 (Tröndle, Moorenweis, Germany) using the iTEM imaging software (EMSIS GmbH, Muenster, Germany).
**[Ref.]** J. Robbin Harris: Negative staining of thinly spread biological particulates, Methods in Molecular Biology 117: Electron Microscopy Methods and Protocols chapter S. 13 - 30

### Size-exclusion high performance liquid chromatography (SE-HPLC)

Formation, relative content and size (molar mass and radius) of VLPs was confirmed by size-exclusion high performance liquid chromatography (SE-HPLC) with ultraviolet (UV), light scattering (LS) and refractive index (RI) detection.

SE-HPLC analysis was performed on an Agilent 1200 series HPLC system (Agilent Technologies Deutschland GmbH, Waldbronn, Germany). The HPLC consisted of a G1312A binary pump, G1379B micro vacuum degasser, G1329A automatic liquid sampler, G1330B automatic liquid sampler thermostat, G1316A thermostated column compartment and a G1314C variable wavelength detector. The Wyatt Technology Europe GmbH instruments miniDAWN TREOS laser photometer with a quasi-elastic light scattering dynamic light scattering module (QELS-DLS detector) and Optilab rEX refractometer were coupled in-line with the SE-HPLC system.

Maximum sample injection volume was 100 µl and samples were thermostated in the autosampler at 23 °C prior to injection. Runs were performed at 25 °C and conducted in triplicates. VLPs were separated by using a TSKgel PWxl guard column (40 × 6 mm, 12 µm particle size, mixed pore size, Tosoh) together with a TSKGel G6000 PWxI analytical column (300 × 7.8 mm, 13 µm particle size, >1000 Å pore diameter, Tosoh Bioscience, Stuttgart, Germany) as stationary phase and 25 mM di-Sodium hydrogen phosphate/125 mM Sodium chloride/1 mM Sodium chloride (pH 7.4) as SE-HPLC eluent (mobile phase) at a flow rate of 0.8 ml/min.

SE-HPLC system control and peak integration from UV-signal for VLP content determination was done with the software ChemStation (version B.04.03, Agilent). The downstream connected Wyatt detectors were controlled by the program ASTRA (version5, Wyatt) and used for characterization of VLPs by measurement of size via multi-angle light scattering (MALS), dynamic light scattering (DLS) and refractive index (RI) detection. Data analysis and calculation of molar mass and radius of VLPs was done with program tools and models of ASTRA, too.

### Capillary gel electrophoresis (CGE)

For purity test of VP1, a CGE-on-a-chip analysis were performed on an Agilent 2100 bioanalyzer (Agilent Technologies Deutschland GmbH; Waldbronn, Germany) in combination with an Agilent Protein 230 Kit (sizing range: 14-230 kDa) and 2100 Expert Software (Kuschel, M., et al. 2002, J Biomol Tech 13(3): 172-178). All reagents and chips were prepared according to the manufacturer's instructions. The gel-dye mix for protein separation was pipetted into the designated reservoirs on a chip and pressed into the microfluidic channels using a chip priming station.

4 µl of each VLP sample and 2 µl of sample buffer were mixed and heated at 95 °C for 5 min. Sample buffer for reducing conditions contained lithium dodecylsulfate, two internal standards and 3.5 % (v/v) of 1 M DTT as a reducing agent. After adding 84 µl of water to each sample-buffer mix, 6 µl of each sample were loaded onto a chip together with and a protein ladder. The chip run results were displayed as a gel-like image, electropherograms and in tabular form. Peak baseline adjusting and peak integration of electropherograms were done automatically and, if necessary, manual adjusting of peak baselines was done on a case-by-case basis. Protein peaks for the desired product (fully VP1 coat protein) and for a product-related substance (*N*-terminally truncated VP1) could be identified and were clearly distinguishable form product- or process-related impurities by comparing migration time in seconds or protein size in kDa, respectively.
**[Ref.]** Kuschel, M., T. Neumann, et al. (2002). "Use of lab-on-a-chip technology for protein sizing and quantitation." J Biomol Tech 13(3): 172-178.

### Results

The H472R amino acid substitution significantly alters the VLP assembly of and improves VLP homogeneity. A single, highly homogenic VLP product was detected in a test batch produced in pilot scale.
SE-HPLC: Chi_NCHR showed much higher VLP content and purity (95.0 %) compared to former Chi and Chi_NC preparations (75-85 %)
CGE, reducing: VP1 degradation of Chi_NCHR remained on the same level (80-85 %), no improvement compared to Chi_NC preparations
TEM: no significant changes, maybe Chi_NCHR's protruding domains are more pronounced than for Chi_NC-VLPs or Chi-VLPs

### EXAMPLE 4

### Comparative immunogenicity of purified GI.4 Chiba NC (wild type) and GI.4 Chiba NCHR mutant version VP1 VLPs in mice using intramuscular (IM) route of delivery.

Recombinant norovirus VLPs were administered two times to BALB/c mice at weeks 0 and 3 in PBS (pH 7.3) IM. Groups were immunized with 10 µg VLP dose of GI.4 Chiba VLPs, either GI.4 Chiba NC (Group I) or GI.4 Chiba NCHR (Group II). All mice were terminated at study week 5. Humoral (antibody) immune responses were analyzed by ELISA-based assays.

### Study animals

BALB/c Ola/Hsd female mice (Envigo, Netherlands) were shipped at ambient temperature to animal facility. Animals were acclimatized one week prior to immunizations and immunized at 7 weeks of age. Health monitoring data summary form was provided with the shipment of the mice. Animal health (clinical signs of illness) and welfare were monitored daily by the staff of the animal facility. All procedures were authorized and performed according to the guidelines of the Finnish National Animal Experiment Board.

### Immunization procedures

The mice were anesthetized with inhalation of isoflurane for the time of immunization and related procedures. Animals were weighted at the beginning of the study and marked with a group tattoo and individually by ear piercing. Test article was administered IM in the caudal tight muscle (50 µl volume) with 0.5 ml insulin syringe (29G×1/2"- 0.33×12 mm).

### Termination procedures and sample collection

Animal weights were recorded at the time of termination. The mice were terminated by anesthetizing mice with 1 mg/kg medetomidine (Dorbene^{®} 1 mg/ml, Laboratorios Syva) and 75 mg/kg ketamine (Ketalar^{®} 50 mg/ml, Pfizer) and collecting the terminal whole blood from axillary (armpit) area. Serum was separated from individually collected whole blood samples and stored at -20°C until used.

### Humoral immune response assays

Titers of antigen-specific IgG in serum were tested by enzyme-linked immunosorbent assay (ELISA) as previously described (Blazevic et al., 2011, Vaccine, 29:81268133; Tamminen et al., 2012, Immunology, 135:89-99). Pig gastric mucin (PGM)-based homologous blocking assay (Lindesmith et al., 2012, J. Virol., 86:873-883) was used to determine the ability of immune sera to block the binding of NoV VLPs to the putative NoV receptors, human histo-blood group antigens (HBGA) as previously described (Uusi-Kerttula et al., 2014, Microbes Infect., 16:472-480).

Both GI.4 Chiba VLPs induced considerable genotype-specific serum IgG response in BALB/c mice when administrated parenterally via IM route with 10 µg dose (Figure 11). No significant difference was observed between individual mice or the two experimental groups (Figure 11). Furthermore, pooled sera of both groups immunized twice with 10 µg GI.4 Chiba VLPs had equally high blocking activity of homologous GI.4 Chiba VLP binding to PGM HBGA receptors (Figure 11). In conclusion, no difference in immunogenicity of GI.4 Chiba NC (Group I) or GI.4 Chiba NCHR (Group II) VLPs was observed when comparing immune responses induced.

### AMINO ACID SEQUENCES

SEQ ID No: 1: Norovirus GI.4 Chiba 407 (Japan, 1987) capsid protein VP1 wild-type sequence (GenBank acc.: BAB18267) 
MMMASKDATPSADGATGAGQLVPEVNTADPIPIDPVAGSSTALATAGQVNLIDPWIINNFVQAPQGE
FTISPNNTPGDVLFDLQLGPHLNPFLSHLSQMYNGWVGNMRVRVVLAGNAFTAGKVIICCVPPGFQS
RTLSIAQATLFPHVIADVRTLDPVEVPLEDVRNVLYHNNDTQPTMRLLCMLYTPLRTGGASGGTDSF
VVAGRVLTCPGPDFNFLFLVPPTVEQKTRPFTVPNIPLKYLSNSRIPNPIEGMSLSPDQTQNVQFQN
GRCTIDGQPLGTTPVSVSQLCKFRGRITSGQRVLNLTELDGSPFMAFAAPAPAGFPDLGSCDWHIEM
SKIPNSSTQNNPIVTNSVKPNSQQFVPHLSSITLDENVSSGGDYIGTIQWTSPPSDSGGANTNFWKI
PDYGSSLAEASQLAPAVYPPGFNEVIVYFMASIPGPNQSGSPNLVPCLLPQEYITHFISEQAPIQGE
AALLHYVDPDTNRNLGEFKLYPGGYLTCVPNSSSTGPQQLPLDGVFVFASWVSRFYQLKPVGTAGPA
RGRLGVRR
SEQ ID No: 2: Norovirus GI.4 Chiba 407 (Japan, 1987) capsid protein VP1 sequence with a single initiator codon and deletion of the two C-terminal arginine residues 
MASKDATPSADGATGAGQLVPEVNTADPIPIDPVAGSSTALATAGQVNLIDPWIINNFVQAPQGEFT
ISPNNTPGDVLFDLQLGPHLNPFLSHLSQMYNGWVGNMRVRVVLAGNAFTAGKVIICCVPPGFQSRT
LSIAQATLFPHVIADVRTLDPVEVPLEDVRNVLYHNNDTQPTMRLLCMLYTPLRTGGASGGTDSFVV
AGRVLTCPGPDFNFLFLVPPTVEQKTRPFTVPNIPLKYLSNSRIPNPIEGMSLSPDQTQNVQFQNGR
CTIDGQPLGTTPVSVSQLCKFRGRITSGQRVLNLTELDGSPFMAFAAPAPAGFPDLGSCDWHIEMSK
IPNSSTQNNPIVTNSVKPNSQQFVPHLSSITLDENVSSGGDYIGTIQWTSPPSDSGGANTNFWKIPD
YGSSLAEASQLAPAVYPPGFNEVIVYFMASIPGPNQSGSPNLVPCLLPQEYITHFISEQAPIQGEAA
LLHYVDPDTNRNLGEFKLYPGGYLTCVPNSSSTGPQQLPLDGVFVFASWVSRFYQLKPVGTAGPARG
RLGV
SEQ ID No: 3: Norovirus GI.4 Chiba 407 (Japan, 1987) capsid protein VP1 sequence with a single initiator codon, deletion of the two C-terminal arginine residues and H472R mutation 
MASKDATPSADGATGAGQLVPEVNTADPIPIDPVAGSSTALATAGQVNLIDPWIINNFVQAPQGEFT
ISPNNTPGDVLFDLQLGPHLNPFLSHLSQMYNGWVGNMRVRVVLAGNAFTAGKVIICCVPPGFQSRT
LSIAQATLFPHVIADVRTLDPVEVPLEDVRNVLYHNNDTQPTMRLLCMLYTPLRTGGASGGTDSFVV
AGRVLTCPGPDFNFLFLVPPTVEQKTRPFTVPNIPLKYLSNSRIPNPIEGMSLSPDQTQNVQFQNGR
CTIDGQPLGTTPVSVSQLCKFRGRITSGQRVLNLTELDGSPFMAFAAPAPAGFPDLGSCDWHIEMSK
IPNSSTQNNPIVTNSVKPNSQQFVPHLSSITLDENVSSGGDYIGTIQWTSPPSDSGGANTNFWKIPD
YGSSLAEASQLAPAVYPPGFNEVIVYFMASIPGPNQSGSPNLVPCLLPQEYITHFISEQAPIQGEAA
LLRYVDPDTNRNLGEFKLYPGGYLTCVPNSSSTGPQQLPLDGVFVFASWVSRFYQLKPVGTAGPARG
RLGV
SEQ ID No:4: Norovirus GI.4 Chiba 407 (Japan, 1987) capsid protein VP1 sequence with a single initiator codon, deletion of the two C-terminal arginine residues, and T39K, T43D, H472R mutations 
MASKDATPSADGATGAGQLVPEVNTADPIPIDPVAGSSKALADAGQVNLIDPWIINNFVQAPQGEFT
ISPNNTPGDVLFDLQLGPHLNPFLSHLSQMYNGWVGNMRVRVVLAGNAFTAGKVIICCVPPGFQSRT
 
LSIAQATLFPHVIADVRTLDPVEVPLEDVRNVLYHNNDTQPTMRLLCMLYTPLRTGGASGGTDSFVV
AGRVLTCPGPDFNFLFLVPPTVEQKTRPFTVPNIPLKYLSNSRIPNPIEGMSLSPDQTQNVQFQNGR
CTIDGQPLGTTPVSVSQLCKFRGRITSGQRVLNLTELDGSPFMAFAAPAPAGFPDLGSCDWHIEMSK
IPNSSTQNNPIVTNSVKPNSQQFVPHLSSITLDENVSSGGDYIGTIQWTSPPSDSGGANTNFWKIPD
YGSSLAEASQLAPAVYPPGFNEVIVYFMASIPGPNQSGSPNLVPCLLPQEYITHFISEQAPIQGEAA
LLRYVDPDTNRNLGEFKLYPGGYLTCVPNSSSTGPQQLPLDGVFVFASWVSRFYQLKPVGTAGPARG
RLGV
SEQ ID No: 5: Norovirus GI.4 Chiba 407 (Japan, 1987) capsid protein VP1 sequence with a single initiator codon, deletion of the two C-terminal arginine residues, and T39D, T43K, H472R mutations 
MASKDATPSADGATGAGQLVPEVNTADPIPIDPVAGSSDALAKAGQVNLIDPWIINNFVQAPQGEFT
ISPNNTPGDVLFDLQLGPHLNPFLSHLSQMYNGWVGNMRVRVVLAGNAFTAGKVIICCVPPGFQSRT
LSIAQATLFPHVIADVRTLDPVEVPLEDVRNVLYHNNDTQPTMRLLCMLYTPLRTGGASGGTDSFVV
AGRVLTCPGPDFNFLFLVPPTVEQKTRPFTVPNIPLKYLSNSRIPNPIEGMSLSPDQTQNVQFQNGR
CTIDGQPLGTTPVSVSQLCKFRGRITSGQRVLNLTELDGSPFMAFAAPAPAGFPDLGSCDWHIEMSK
IPNSSTQNNPIVTNSVKPNSQQFVPHLSSITLDENVSSGGDYIGTIQWTSPPSDSGGANTNFWKIPD
YGSSLAEASQLAPAVYPPGFNEVIVYFMASIPGPNQSGSPNLVPCLLPQEYITHFISEQAPIQGEAA
LLRYVDPDTNRNLGEFKLYPGGYLTCVPNSSSTGPQQLPLDGVFVFASWVSRFYQLKPVGTAGPARG
RLGV
SEQ ID No: 6:gb:AGJ52175|GI Hu/GI/HuzhouNll/2008/CHN 
MMMASKDAPTSPDGASGAGQLVPEANTAEQISMDPVAGASTAVATAGQVNMIDPWIFNNF
VQAPQGEFTISPNNTPGDILFDLQLGPHLNPFLAHLSQMYNGWVGNMRVRILLAGNAFTA
GKIIICCVPPGFDARILTIAQATLFPHLIADVRTLEPVELPLEDVRNVLYHNSSQPQPTM
RLVAMLYTPLRTGGGSGGTDAFVVAGRVLTCPAPDFSFLFLVPPSVEQKTRVFSVPNIPL
KDLSNSRVPVPIQGMFMSPDVNQSVQFQNGRCQIDGQLQGTTPVSLSQLCKIRGKTSSNA
RVLNLSEVDGTPFIPLESPAPVGFPDLGGCDWHVNFSFQTQDRDPSQSVTFATNDASFVP
YLGSVSPHNGEGFQAGDIIGSLGWISAPSDNSQFNVWAIPKYGSSLQMSPILLLLCSPRL
WEVILYFYSTFPGSGQPSQLQVPCLLPQEFITHFCNEQAPIAGEAALLHYVDPDTGRNLG
EFKLYPDGFMTCVPNSVSSGPQTLPINGVFVFVSWVSRFYQLKPVGTASAARRLGLRRI
SEQ ID No: 7:gb:AFNO6736|GI Hu/GI/E8/UG/1976 
MMMASKDAPTNMDGTSGAGQLVPEANTAEPISMDPVAGAATAVATAGQINMIDPWIMSNF
VQAPQGEFTVSPNNTPGDVLFDLQLGPQLNPFLAHLAQMYNGWVGNMRVKVLLAGNAFTA
GKIIISCIPPGFTSQNISIAQMTMFPHVIADVRVLEPIEIPLEDVRNVLFHTNDNRPTMR
LVCMLYTPLRANGSSSGTDPFVIAGRVLTCPDSNFSFLFLVPPNVEQKTRPFSVPNIPLN
TLSNSRVPSLIKSMTISRDQNQIIQFQNGRVTLDGQLQGTTPTSVSQLCKIRGTTYHATG
GNGINLTELNGEPYHAFESPAPIGFPDLGGCDWHLTATPTQAFNDGAKVVRLSVTQGAAF
APHLGTIHYTTTDHDYDPNTSIICTLDWLSQTTGQNNVDPWQIPTYGSTLTEAAQLAPPI
FPPGFGETLVFFLSDFPISNGKNGLSVPCTLPQEFVTHFVNEQAPIRGEAALLHYVDPDT
HRNLGEFKLYPEGFMTCVPNTSGGGPQTLPINGVFVFVSWVSRFYQLKPVGTAGAARRLG
IRRS
SEQ ID No: 8:gb:AUF81820|GI Hu/ETH/2016/P15 
MMMASKDAPTNMDGTSGAGQLVPEANTAEPISMEPVAGAATAAATAGQVNMIDPWIMNNY
VQAPQGEFTISPNNTPGDILFDLQLGPHLNPFLSHLAQMYNGWVGNMKVKVLLAGNAFTA
GKIIISCIPPGFAAQNISIAQATMFPHVIADVRVLEPIEIPLEDVRNVLFHNNDNTPTMR
LVCMLYTPLRASGSSSGTDPFVIAGRVLTCPSPDFSFLFLVPPNVEQKTKPFSVPNLPLN
ILSSSRVPSLIKSMMISRDHGQMVQFQNGRVTLDGQLQGTTPTSASQLCKIRGSVFHANG
GNGYNLTELDGSPYHAFESPAPIGFPDLGECDWHMEASPTIQFDTGDVIKQINVKQESAF
APHLGTVQADGLDGVSANTNMIAKLGWVSPVSDGHRRDVDPWVIPRYGSTLTEAAQLAPP
IYPPGFGEAIVFFMSDFPIAHGTNGLSVPCTIPQEFVTHFVNEQAPTRGEAALLHYLDPD
THRNLGEFKLYPDGFMTCVPNSSGSGPQTLPINGVFVFVSWVSRFYQLKPVGTAGPARRL
GIRRS
SEQ ID No: 9:gb:BAV21674|GI Hu/GI/46-2/Tokyo/1977/JPN 
MMMASKDAPSNMDGTSGAGQLVPEANTAEPINMESVVGAATATATAGQVNLIDPWIMNNY
VQAPQGEFTISPNNTPGDVLFDLQLGPHLNPFLSHLSRMYNGWVGNMKVRVMLAGNAFSA
GKIIICCIPPGFTSQSISIAQATMFPHVIADVRVLEPIDVPLDDVRNVLFHNNDNAQTMR
LLCMLYTPLRTGASSSGSDPFVIAGRVLTCPTQDFNFLFLVPPDVEQKTKPFSVPNIPLN
LMSNSRVPALIDGMTVSSDQNQVVQFQNGRVTLDGQLQGTTAVSASCVAKIRGRIFSNAS
HYGINLTEVDGTQYHAFDSPAPLGFPDFGNCDWHVTGTKASQGDLQTDNPTISGTIKSYE
SSFAPHLGTVRIEGDDNELARFNGKDVLLNLTWFSQRNGSQLNLWTIPSYGSNLTEASQL
APPIYPPGFGEAIVYFTSTFPAISRPSVPCTMPQEFVSHFVNEQAPTRGEAALLHYLDPD
THRNLGEFKMYPEGFFTCVPNAGGSGPQTLPINGVFVFVSWVSRYYQLKPVGTVGMTRRL
GLMKQ
SEQ ID No: 10:gb:NP_056821GI.1 GI/Human/United States/Norwalk/1968 
MMMASKDATSSVDGASGAGQLVPEVNASDPLAMDPVAGSSTAVATAGQVNPIDPWIINNF
VQAPQGEFTISPNNTPGDVLFDLSLGPHLNPFLLHLSQMYNGWVGNMRVRIMLAGNAFTA
GKIIVSCIPPGFGSHNLTIAQATLFPHVIADVRTLDPIEVPLEDVRNVLFHNNDRNQQTM
RLVCMLYTPLRTGGGTGDSFVVAGRVMTCPSPDFNFLFLVPPTVEQKTRPFTLPNLPLSS
LSNSRAPLPISSMGISPDNVQSVQFQNGRCTLDGRLVGTTPVSLSHVAKIRGTSNGTVIN
LTELDGTPFHPFEGPAPIGFPDLGGCDWHINMTQFGHSSQTQYDVDTTPDTFVPHLGSIQ
ANGIGSGNYVGVLSWISPPSHPSGSQVDLWKIPNYGSSITEATHLAPSVYPPGFGEVLVF
FMSKMPGPGAYNLPCLLPQEYISHLASEQAPTVGEAALLHYVDPDTGRNLGEFKAYPDGF
LTCVPNGASSGPQQLPINGVFVFVSWVSRFYQLKPVGTASSARGRLGLRR
SEQ ID No: 11:gb:ARC53064|GI.2 Hu/GI.2/Kaohsiung/16-AF-2/2016/TW 
MMMASKDAPQSADGASGAGQLVPEVNTADPLPMEPVAGPTTAVATAGQVNMIDPWIVNNF
VQSPQGEFTISPNNTPGDILFDLQLGPHLNPFLSHLSQMYNGWVGNMRVRILLAGNAFSA
GKIIVCCVPPGFTSSSLTIAQATLFPHVIADVRTLEPIEMPLEDVRNVLYHTNDNQPTMR
LVCMLYTPLRTGGGSGSSDSFVVAGRVLTAPSSDFSFLFLVPPTIEQKTRAFTVPNIPLQ
TLSNSRFPSLIQGMILSPDASQVVQFQNGRCLIDGQLLGTTPATSGQLFRVRGKINQGAR
TLNLTEVDGKPFMAFDSPAPVGFPDFGKCDWHMRISKTPNNTSSGDPMRSVSVQTNVQGF
VPHLGSIQFDEVFNHPTGDYIGTIEWISQPSTPPGTDINLWEIPDYGSSLSQAANLAPPV
FPPGFGEALVYFVSAFPGPNNRSAPNDVPCLLPQEYITHFVSEQAPTMGDAALLHYVDPD
TNRNLGEFKLYPGGYLTCVPNGVGAGPQQLPLNGVFLFVSWVSRFYQLKPVGTASTARGR
LGVRRI
SEQ ID No: 12:gb:AFN06739|GI.3 Hu/GI.3/B8/CF/1977 
MMMASKDAPTNMDGTSGAGQLVPEANTAEPISMEPVAGAATAAATAGQVNMIDPWIMNNY
VQAPQGEFTISPNNTPGDILFDLQLGPHLNPFLSHLAQMYNGWVGNMKVKVLLAGNAFTA
GKIIISCIPPGFASQNISIAQATMFPHVIADVRVLEPIEVPLEDVRNVLFHNNDNTPTMR
LVCMLYTPLRASGSSSGTDPFVIAGRVLTCPSPDFSFLFLVPPNVEQKTKPFSVPNLPLN
VLSNSRVPSLIKSMMVSQDHGQMVQFQNGRVTLDGQLQGTTPTSASQLCKMRGTVYHASG
GQGLNLTEIDGTPYHAFESPAPIGFPDIGDSDWHINASPATTFDSGESIKRLDMEQGSSF
APHLGTVHYTNADYPANTDLICSLEWLSPPSGGTPNKVNPWTIPRYGSTLTEAAQLAPPI
YPPGFGEAIVFFMSDFPIANGQDGLKVPCTIPQEFVTHFVNEQAPTRGEAALLHYVDPDT
HRNLGEFKLYPEGFMTCVPNSSGSGPQTLPINGVFTFVSWVSRFYQLKPVGTTGPVRRLG
IRRS
SEQ ID No: 13:gb:AOO95034|GI.3 15-EN-3/2015 
MMMASKDAPTNMDGTSGAGQLVPEANTAEPISMEPVAGAATAAATAGQVNMIDPWIMNNY
VQAPQGEFTISPNNTPGDILFDLQLGPHLNPFLSHLAQMYNGWVGNMKVKVLLAGNAFTA
GKIIISCIPPGFASQNISIAQATMFPHVIADVRVLEPIEVPLEDVRNVLFHNNDNSPTMR
LVCMLYTPLRASGSSSGTDPFVIAGRVLTCPSPDFSFLFLVPPNVEQKTKPFSVPNLPLN
TLSNSRVPSLINAMMISRDHGQMVQFQNGRVTLDGQLQGTTPTSLSQLCKIRGKVFLASG
GNGLNLTELDGSAYHAFESPAPIGFPDIGDCDWHMNATATSNFTGSNDEHQILVKQESTF
 
APHLGHVQADHLPEVANTDLMVSLSWISPVSDQHRRDVDPWVIPRYGSTLTEAAQLAPPI
YPPGFGEAIVFFMSDFPVVSGVNGMRIPCTLPQEYVAHFVNEQAPTRGEAALLHYVDPDT
HRNLGEFKIYPEGFMTCVPNSSGTGPQTLPINGVFTFVSWVSRFYQLKPVGTAGPARRLG
IRRS
SEQ ID No: 14:qb:AEY77031IGI.4 Hu/GI.4/S14/2008/LillaEdet/Sweden 
MMMASKDATPSADGATGAGQLVPEVNTADPIPIDPVAGSSTALATAGQVNLIDPWIINNF
VQAPQGEFTISPNNTPGDVLFDLQLGPHLNPFLSHLSQMYNGWVGNMRVRVVLAGNAFTA
GKVIICCVPPGFQSRTLSIAQATLFPHVIADVRTLDPVEVPLEDVRNVLYHNNDTQPTMR
LLCMLYTPLRTGGASGGTDSFVVAGRVLTCPGPDFNFLFLVPPTVEQKTRPFTVPNIPLK
YLSNSRIPNPIEGMSLSPDQTQNVQFQNGRCTIDGQPLGTTPVSVSQLCKFRGRITSGQR
VLNLTELDGSPFMAFAAPAPAGFPDLGSCDWHIEMSKIPNSSTQNNPIVVNSVKPNSQQF
VPHLSSITLDDNVSSGGDYIGTIQWTSPPSDSGGANTNFWKIPDYGSSLAEASQLAPAVY
PPGFNEVIVYFMASIPGPNQSGSPNLVPCLLPQEYITHFISEQAPIQGEAALLHYVDPDT
NRNLGEFKLYPGGYLTCVPNSSSTGPQQLPLDGVFVFASWVSRFYQLKPVGTA
SEQ ID No: 15:gb:BAA82106|GI.4 Hu/Chiba407/87/JP 
MMMASKDATPSADGATGAGQLVPEVNTADPIPIDPVAGSSTALATAGQVNLIDPWIINNF
VQAPQGEFTISPNNTPGDVLFDLQLGPHLNPFLSHLSQMYNGWVGNMRVRVVLAGNAFTA
GKVIICCVPPGFQSRTLSIAQATLFPHVIADVRTLDPVEVPLEDVRNVLYHNNDTQPTMR
LLCMLYTPLRTGGASGGTDSFVVAGRVLTCPGPDFNFLFLVPPTVEQKTRPFTVPNIPLK
YLSNSRIPNPIEGMSLSPDQTQNVQFQNGRCTIDGQPLGTTPVSVSQLCKFRGRITSGQR
VLNLTELDGSPFMGFGAPAPAGFPDLGSCDWHIEMSKIPNSSTQNNPIVTNSVKPNSQQF
VPHLSSITLDENVSSGGDYIGTIQWTSPPSDSGGANTNFWKIPDYGSSLAEASQLAPAVY
PPGFNEVIVYFMASIPGPNQSGSPNLVPCLLPQEYITHFISEQAPIQGEAALLHYVDPDT
NRNLGEFKLYPGGYLTCVPNSSSTGPQQLPLDGVFVFASWVSRFYQLKPVGTAGPARGRL
GVRR
SEQ ID No: 16:gb:A0095019|GI.5 15-EN-8/2015/GI.5 
MMMASKDATPSADGANGAGQLVPEVNNAEPLPLDPVAGASTALATAGQVNMIDPWIFNNF
VQAPQGEFTISPNNTPGDILFDLQLGPHLNPFLAHLSQMYNGWVGNMRVRVILAGNAFTA
GKVIICCVPPGFQSRTLSIAQATLFPHIIADVRTLEPIEIPLEDVRNTLYHTNDNQPTMR
LLCMLYTPLRTGGGSGGTDAFVVAGRVLTSPSPDFNFLFLVPPTVEQKTRPFSVPNIPLQ
LLSNSRVPNLIQSMVPSPDQAQNVQFQNGRCTTDGQLLGTTPVSVSQILKFRGKVSAGSK
VINLTELDGSPFLAFEAPAPTGFPDLGTSDWHIEMSLNSNSQSSGNPILLRDIQPNSSDF
VPHLGSVAVTTAIDTAGDYTGTIQWTSQPSNVTPVPDVNFWTIPQYGSNLAEASQLAPVV
YPPGFGEAIVYFMSPIPGPNTAHKPNLVPCLLPQEFVTHFVSEQAPSMGEAALVHYVDPD
TNRNLGEFKLYPEGFITCVPNGTGPQQLPLNGVFVFASWVSRFYQLKPVGTANSARGRLG
VRR
SEQ ID No: 17:gb:BAU16307|GI.5 Hu/Jp/2002/GI.5/OC020180 
MMMASKDATPSADGANGAGQLVPEVNNAEPLPLDPVAGASTALATAGQVNMIDPWIFNNF
VQAPQGEFTISPNNTPGDILFDLQLGPHLNPFLAHLSQMYNGWVGNMRVRVILAGNAFTA
GKVIICCVPPGFQSRTLSIAQATLFPHVIADVRTLEPLEIPLEDVRNTLYHNNDSQPTMR
LLCMLYTPLRTGGGSGGTDAFVVAGRVLTCPSPDFNFLFLVPPTVEQKTRPFSVPNIPLQ
NLSNSRVPSLIQSMVLSSDHAQTVQFQNGRCTTDGHLLGTTPVSSGQLMKFRGKVTPGSK
VLNLTELDGSPFLAFEPPAPAGFPDLGKCDWHIEMSLYQVNNQDNPIVLRAIEPNSSSFV
PHLGSVSFNQNVDAAGDYVCTIQYTSPPSNSHDADVDFWSIPDYGSNLAEASQLAPVVYP
PGFGEAIVYFMSRVPGWNRTNRLNLVPCLLPQEFIGHFVSEQAPAIGEAALLHYVDPDTN
RNLGEFKLYPEGFITCVPNGTGPQQLPLNGVFVFSSWVSRFYQLKPVGTASSARGRLGIR
R
SEQ ID No: 18:gb:BAU16303|GI.6 Hu/Jp/2013/GI.6/s130147 
MMMASKDVPTSPDGASGAGQLVPEVNTADQISMDPVAGASTAVATAGQVNMIDPWIFNNF
VQAPQGEFTISPNNTPGDILFDLQLGPHLNPFLAHLSQMYNGWVGNMRVRILLAGNAFTA
 
GKIIICCVPPGFDARILTIAQATLFPHLIADVRTLEPVELPLEDVRNVLYHNSSQPQPTM
RLVAMLYTPLRTGGGSGGTDAFVVAGRVLTCPAPDFSFLFLVPPSVEQKTRVFSVPNIPL
KDLSNSRVPTLIQGMFVSPDVNQSVQFQNGRCQIDGQLQGTTPVSLSQLCKIRGKTSSNT
RVLNLSEVDGTPFVPLESPAPVGFPDIGGCDWHVGFTFEARDQGPSQNVTFATNDSSFVP
YLGSISPHNGDGFHSGDIIGSLDWISAPSDGSALDVWSIPKYGSSLPDVTHLAPAVFPPG
FGEVILYFHSKFPGSGPTDKLRVPCLMPQEFITHFCDEQAPIAGEAALLHYVDPDAGRNL
GEFKLYPDGFMTCVPNSISSGPQTLPINGVFVFVSWVSRFYQLKPVGTASMARRLGLRRI
SEQ ID No: 19:gb:APA31976|GI.7 Hu/USA/2011/GI.P7_GI.7/CS5567 
MMMASKDAPSNMDGTSGAGQLVPEVNAAEPLPLEPVVGAATAVATAGQVNLIDPWIMNNF
VQAPEGEFTISPNNTPGDILFDLQLGPHLNPFLQHLSQMYNGWVGNMRVRVMLAGNAFTA
GKIIICCVPPGFASQNISIGQATMFPHVIADVRVLEPIEIPLDDVRNVLFHTNESRPTMR
LLCMLYTPLRAGGASSGTDPFVIAGRVLTCPSPDFNFLFLVPPSVEQKTRQLTVPNIPLN
NLANSRVPAMINKMTVSTDQSQVVQFQNGRCTLEGQLLGTTPVSASQVARIRGKVFSTAS
GKGLNLTELDGTPYHAFESPAPLGFPDIGACDWHVSTFKVDQNLSGDPMSRLDIKQNAPF
APHLGSIEFTSDQEPTGDQLGTLAWVSPSTSGARVDPWKIPSYGSTVTESTHLAPPIFPP
GFGEAIVYFMSDFPIVSGNTAQVPCTLPQEFVSHFVEQQAPIRGEAALLHYVDPDTHRNL
GEFKLYPDGFITCVPNTGGGPQNLPINGVFVFSSWVSRYYQLKPVGTAGPARRLGVRRV
SEQ ID No: 20:gb:AKM20815|GI.8 Hu/CHN/2008/GI.P8 GI.8/Huzhou/N10 
MMMASKDAPTNMDGTSGAGQLVPEANTAEPLPIKPVAGAATAVATAGQVNMIDPWIMNNF
VQAPQGEFTISPNNTPGDILFDLQLGPHLNPFLAHLSRMYNGWVGNMQVRIMLAGNAFSA
GKIIVCCIPPGFSSQSISIAQATMFPHVIADVRVLEPIDVPLDDVRNVLFHNNDNPQTMR
LLCMLYTPLRSGGTSSGTDPFVIAGRVLTCPTPDFSFLFLVPPDIEQRTKPFSVPNIPMN
LMSNSRVSMLIDGMMVSNDQNQVPQFQNGRVTLDGQLQGTTTVSAACVARMRGRIFNNNG
NYGVNLTELDGNPYHAFDSPAPLGFPDFGNCDLHMTFVKINPNELSSGDPSGKVVIRSYD
ATFAPHLGTVKLENDDELARFVGKEVVLELTWVSNREGATLNLWAVPNYGSSLTQASQLA
PPIYPPGFGEAIVYFTSTFPTVSNPKVPCTLPQEFVSHFVNEQAPTRGDAALLHYVDPDT
HRNLGEFKMYPEGYMTCVPNAGGGPQTLPINGVFVFISWVSRYYQLKPVGTAGAARRLGL
RRS
SEQ ID No: 21:qb:APA31982IGI.9 Hu/USA/2016/GI.P9 GI.9/SC6350 
MMMASKDATSNMDGTSGAGQLVPENNNTSEPINMEPVAGAVTAAATAGQVNMIDPWIMNN
YVQAPQGEFTISPNNTPGDILFDLQLGPHLNPFLAHLSQMYNGWVGNMKVRVVLAGNAFS
AGKIIVCCIPPGFSAPNISIAQATMFPHVIADVRVLEPIDIPLDDVRNVLFHNNDNGNQT
MRLLCMLYTPLRSGGTSSGTDPFVIAGRVLTCPTPDFNFLFLVPPTVEQKTKQFSVPNLP
LNVMSNSRVPSLLNAMVVSPDQAQVVQFQNGRCTLDGQMLGTTTVSASCVARFRGKTFQA
PDNRLGINLAEISGEPYHAFESPAPLGFPDFGDGDWHVTATKVTPSQLEANDPVVMGNVQ
PYNPQFAPHLGTLVVENPTPDNVTTGTDLLFNITWLSNRANNRFNPWVIPNYGSTLTEAA
QLAPSIFPPGFGETIVYFNSTFPAVGATTHAAIPCLLPQEFVAHFVNEQAPIRGEAALLH
YIDPDTHRNLGEFKIYPEGFVTCVPNVGGTGPQSLPTNGIFVFVSWVSRYYQLKPVGTAG
QARRLGFRRV
SEQ ID No: 22:gb:AGL98416|GII Hu/GII/T091/TN/1976 
MKMASNDANPSDGSAANLVPEVNNEVMALEPVVGAAIAAPVAGQQNVIDPWIRNNFVQAP
GGEFTVSPRNAPGEILWSMPLSPELNPYLSHLSRMYNGYAGGFEVQVILAGNAFTAGKII
FAAIPPNFPTDGLSPSQVTMFPHIIVDVRQLEPVLIPLPDVRNNFYHYNQANDSTLKLIA
MLYTPLRANNAGDDVFTVSCRVLTRPSPDFDFIFLVPPTVESRTKPFTVPILTVDEMTNS
RFPTPLEKLHTGPNNSIVVQPQNGRCTIDGVLLGTTQLSTVNICNFRGSTTRAGQSHAYT
MNLVSQNWNNYDPTEEIPAPLGTPDFVGKIVGMLSQTTRENSSTRAHKATVSTGDAHFTP
KSGSVLFTTDTDDLQNGQNTRFTPVGVAQDGEPHQNEPQQWRLPNYSGTPGHNVHLAPPV
 
APTFPGEQLLFFRSTMPGCGGYPNMDLCLLPQEWVRHFYQEAAPAQSDVALLRFVNPDT
GRVLFECKLHKAGYITVSHTGPYDLVIPPNGYFRFDSWVNQFYALAPMGTGTGRRRAL
SEQ ID No: 23:gb:AUD54969|GII Hu/PE/2012/GII.PNA1-GII.NA1/Loreto1041 
MKMASNDAAPSNDGAAGLVPEINNETMALEPVAGGAIAAPLTGQTNFIDPWIRNNYVQAP
NGEFTVSPRNSPGEILLNLELGPELNPFLAHLSRMYNGYAGGIDVQVIMAGNAFTAGKII
FAAVPPHFPIDNISPPQITMFPHIIIDVRTLEPVNIPLPDVRNSFFHYSQNNEPRMRLLA
MLYTPLRSNGSADDVFTVSCRVLTKPSADFEFNYLVPPTVESRTKPFTIPILTIGEMTNS
RFPVAIDMLHTSPTDNFIVQPQNGRCTLDGELQGTTQLVTSNICAFRGSISGHENNGDQH
QWHFSITNPNGTPFDPTEDVPAPLGTPDFKGQLYGVISQRNREGSPGNGNQKANRSHEGV
ISTVAPRFTPKLGSVMIGTWTTDDIQDQPSRFTPVGLNDDDNYKQWELPNYSGALTLNMG
LAPSVFPTYPGEQLLFFRSYIPMKGGYGSPYIDCLIPQEWISHFYQESAPSQTDVALIRY
VNPDTGRVLFEAKLHRQGYITVAKTGDSPINVPANGYFRFDSWVSQFYSLAPMGTGNGRR
RIQ
SEQ ID No: 24:gb:AU028670|GII 768/2002/TUN 
MRMASSDAPVSGTDGAAGLVPESQQEVLPLEPVAGVQLAAPVAGQSNIIDPWIRMNFVQA
PAGEFTVSPRNAPGEVLIDLELGPELNPYLNHLARMYNGYVGGMEVEVVLAGNAFTAGKI
LFAAVPPSFPTHGISAAQATMLPHVIVDVRQLEPVRLPLPDVRNVMFHFCQENKEPRMRI
VAILYTPLRANGAGDDVFTVSCRVLTRPSPDFDFIFLVPPSVESKLKQFTLPNLQPNEMT
NSRFPTGITQLYTSPNTNLVVQFQNGRCLLDGTLLGTTPVRAADICSFRGVTSTEVDATD
SPRVAGSHRIMVQLKEPDGEEFSPTGPNPAPVGTPDFQAAIFGTLSQRNTGGTGQNSNRA
HFAYFYTRNPTFAPGIGTVVFSFDTTDFQNRQPTKFSPSGVFDDDSSEPFNQFSLPYYNG
SLGAVDAGKLAPPVAPNYPGEQILYFRGNIPFKGGYGEGEIDSLLPQEWITHFYAEQAPT
QGDAALLRYYNPDTGRVLFECKLHREGFITINYTGSNALAVPVNGVFRFEGWVNKFYTLT
PMGNGNGRRGRRREL
SEQ ID No: 25:gb:BAJ25074|GII Hu/OC07118/2007/JP 
MKMASNDANPSSDGSANLVPEISNEVMALEPVAGAAIAAPVAGQQNIIDPWIRNNFVQAP
GGEFTVSPRNAPGEVLLNLPLSPDINPYLAHLSRMYNGYAGGVEVEVVLAGNAFTAGKII
FAAVPPNFPPENLSPSQITMLPHIIVDVRQLEPVRIPLPDVRNNFYHYSRENDSTLRLIA
MLYTPLRANNAGDDVFTVSCRVLTRPSPDFDFIFLVPPTVESKSKPFTIPMLTIEEMTNS
RFPAPLELMTTGPSHDIVVQPQNGRCTIDGVLLGTTQLSPVNICSLRGVPTKRTNGNDNC
FVQLENPNGSAYDPTEDIPAVLGSPDFVGELYGTITQRSSDNSTRAHPFTLNTGSPRYTP
KIGSVDIRVTDVSDLQDHDPVKLTPVGLSGDRGSIHQWQLPNYSGVATHNMHLAPSVAPL
FPGEQILFFRSTVPGCGGYPNSNIDCLIPQEWVQHFYQEGAPARTDVALLRFINPDTGRV
LFECKLHKHGFITVAYSGNHDLVMPPNGYFRFESWVNQFHTLAPMGTGSGRRRIQ
SEQ ID No: 26:gb:AII73762|GII Hu/JP/2011/GII/Yuzawa/Gira2HS 
MKMASKDASPSTDGTANLVPESQQEVLALQPVAGAQIAAPVAGQFNVIDPWIYQNFVQAP
EGEFTVSPRNSTGEILMNLELGPQLNPYLAHLARMYNAYAGGFEVQVLLAGNAFTAGKII
VCAVPPNFPLQNISAAQATQLPHVVVDVRQLEPVVLPLPDVRAGFYHYNQVEESRMRLVA
ILYTPLRTNSAGDDAFTVSCRILTRPAPDFSFFFLIPPTIESKTTPFTLPRLPISEMTNS
RFPLVIKGMVVDPNLPLQANFQNGRITLDGELQGTTLPTSTSIGRISGTHMSSTPSRIIQ
HEDSGDSTQPRVFNPVWMDLTENNWTEFQPFNDQPAPLGCPDFKAKILGTLIRQPNNGSY
YFDAYLDTRQHGTFAPYTGHAAVHSDQQAGHLAQGYKIQFSPTGIESDQNTDLNQLPDYG
GAMTVSKGLAPAAAPDFPGEMILYFVSDMPVRNPNGERRDTEILCLLPQEMVTHFYEQQA
PSQGDVALVRYINAETGRVMFEGKLHRNGFFTVSATARTLIVPDGYFRFDSWVNRFYTLS
PMGTGNGRRRARMLE
SEQ ID No: 27:gb:ASW22508|GII.1 Hu/IDN/ITD11-3/2015/GII.Pg/GII.1 
MKMASNDVAPSNDGAAGLVPEVSNETMALEPVAGASIAAPLTGQNNVIDPWIRMNFVQAP
NGEFTVSPRNSPGEVLLNLELGPELNPFLAHLARMYNGYAGGVEVQVLLAGNAFTAGKLV
FAAIPPHFPLENLSPGQITMFPHVIIDVRTLEPVLLPLPDVRNNFFHYNQQPEPRMRLVA
 
MLYTPLRSNGSGDDVFTVSCRVLTRPSPDFDFNYLVPPTVESKTKPFTLPILTIGELSNS
RFPVPIDELYTSPNEGVVVQPQNGRSTLDGELLGTTQLVPSNICALRGRINAQVPDDHHQ
WNLQVTNANGTSFDPTEDVPAPLGTPDFLANIYGVTSQRNPDNTCRAHDGVLATWSPKFT
PKLGSVVLGTWEESDLDLNQPTRFTPVGLYDTNHFDQWILPNYSGRLTLNMNLAPSVAPL
FPGEQILFFRSHIPLKGGTSNGAIDCLLPQEWIQHFYQESAPSPTDVALIRYTNPDTGRV
LFEAKLHRQGFITVANSGSRPIVVPPNGYFRFDSWVNQFYSLAPMGTGNGRRRVQ
SEQ ID No: 28:gb:BBB86933|GII.2 Hu/GII/JP/2004/GII.P2_GII.2/Tochigi-86 
MKMASIDAAPSTDGAAGLVPESNNEVMALEPVAGAALAAPVTGQTNIIDPWIRANFVQAP
NGEFTVSPRNAPGEVLLNLELGPELNPYLAHLARMYNGYAGGMEVQVMLAGNAFTAGKLV
FAAVPPHFPVENLSPQQITMFPHVIIDVRTLEPVLLPLPDVRNNFFHYNQKDDPKMRIVA
MLYTPLRSNGSGDDVFTVSCRVLTRPSPDFDFTYLVPPTVESKTKPFTLPILTLGELSNS
RFPVSIDQMYTSPNEVISVQCQNGRCTLDGELQGTTQLQVSGICAFKGEVTAHLHDNDHL
YNVTITNLNGSPFDPSEDIPAPLGVPDFQGRVFGVISQRDKHNSPGHNEPANRGHDAVVP
TYTSQYTPKLGQIQIGTWQTDDLTVNQPVKFTPVGLNDTEHFNQWVVPRYAGALNLNTNL
APSVAPVFPGERLLFFRSYIPLKGGYGNPAIDCLLPQEWVQHFYQEAAPSMSEVALVRYI
NPDTGRALFEAKLHRAGFMTVSSNTSAPVVVPANGYFRFDSWVNQFYSLAPMGTGNGRRR
VQ
SEQ ID No: 29:gb:AUD54972|GII Hu/PE/2013/GII.PNA2-GII.NA2/Loreto1257 
MKMASNDAAPSNDGAAGLVPEINNETMALEPVAGGAIAAPLTGQTNFIDPWIRGNYVQAP
NGEFTVSPRNSPGEILLNLELGPELNPFLAHLSRMYNGYAGGIDVQVIMAGNAFTAGKII
FAAVPPHFPVENISPPQITMFPHIIVDVRTLEPINIPVPDVRNNFFHYNQDRDSRMRLVA
MLYTPLRSNGSTDDVFTVSCRVLTKPSADFEFNYLVPPTVESRTKPFSIPILTIGEMTNS
RFPLPIDMLYTSPTENLVVQPQNGRCTTEGELLGTTQLVTPSICSLRGAITGHEGNDDDH
KWHMTVTSPNGAAFDPTEDVPAPLGTPDFTGDIYGVLSQRDRNINPGQTAPANRAHEAVV
STRSNKFTPKLGSVMIATWETTDVLQQPTKFTPVGLESPNHYNQWQLPNYSGALTLNMGL
APSVFPTYPGEQILFFRSFIPLKGGYGNSAIDCLVPQEWIQHFYQESAPSQTDVALIRYV
NPETGRVLFEAKLHRQGFITVAKTGDSPINVPANGYFRFDSWVNPFYSLAPMGTGNGRRR
NQ
SEQ ID No: 30:gb:BAK43275|GII.3 Hu/Tokyo/10-1105/2010/JPN 
MKMASNDAAPSNDGAAGLVPEINNEAMALEPVAGAAIAAPLTGQQNIIDPWIMNNFVQAP
GGEFTVSPRNSPGEVLLNLELGPEINPYLAHLARMYNGYAGGFEVQVVLAGNAFTAGKII
FAAIPPNFPIDNLSAAQITMCPHVIVDVRQLEPVNLPMPDVRNNFFHYNQGSDSRLRLVA
MLYTPLRANNSGDDVFTVSCRVLTRPSPDFSFNFLVPPTVESKTKPFTLPILTISEMSNS
RFPVPIDSLHTSPTENIVVQCQNGRVTLDGELMGTTQLLPSQICAFRGVLTRSTSRASDQ
ADTATPRLFNYYWHIQLANLNGTPYDPAEDIPGPLGTPDFRGKVFGVACQRNPDCTTRAH
EAKVDTTAGRFTPKLGSLEISTESGDFDQNQPTRFTPVGIGVDHEADFQQWSLPDYSGQF
THNMNLAPAVAPNFPGEQLLFFRSQLPSSGGRSNGILDCLVPQEWVQHFYQESAPAQTQV
ALVRYVNPDTGRVLFEAKLHKLGFMTIAKNGDSPITVPPNGYFRFESWVNPFYTLAPMGT
GNGRRRIQ
SEQ ID No: 31:gb:BAG70446|GII.4 Aomori2/2006/JP 
MKMASSDANPSDGSTANLVPEVNNEVMALEPVVGAAIAAPVAGQQNVIDPWIRNNFVQAP
GGEFTVSPRNAPGEILWSAPLGPDLNPYLSHLARMYNSYAGGFEVQVILAGNAFTAGKII
FAAVPPNFPTEGLSPSQVTMFPHIIVDVRQLEPVLIPLPDVRNNFYHYNQSNDPTIKLIA
MLYTPLRANNAGDDVFTVSCRVLTRPSPDFDFIFLVPPTVESRTKPFSVPILTVEEMTNS
RFPIPLEKLFTGPSSAFVVQPQNGRCTTDGVLLGTTQLSPVNICTFRGDVTHIAGTQEYT
MNLASQNWNNYDPTEEIPAPLGTPDFVGKIQGVLTQTTRRDGSTRGHKATVSTGSVHFTP
KLGRIQFSTDTSNDFETGQNTRFTPVGVVQDGSTTHQNEPQQWVLPNYSGRDSHNVHLAP
AVAPSFPGEQLLFFRSTMPGCSGYPNMNLDCLLPQEWVQHFYQEAAPAQSDVALLRFVNP
DTGRVLFECKLHKSGYVTVAHTGQHDLVIPPNGYFRFDSWVNQFYTLAPMGNGTGRRRAL
SEQ ID No: 32:gb:AND99841|GII.4 32-15 
MKMASSDANPSDGSTASLVPEVNNEVMALEPVVGAAIAAPVAGQQNVIDPWIRNNFVQAP
GGEFTVSPRNAPGEILWSAPLGPDLNPYLSHLARMYNGYAGGFEVQVILAGNAFTAGKII
FAAVPPNFPTEGLSPSQVTMFPHIIVDVRQLEPVLIPLPDVRNNFYHYNQSNDSTIKLIA
MLYTPLRANNAGDDVFTVSCRVLTRPSPDFDFIFLVPPTVESRTKPFSVPILTVEEMTNS
RFPIPLEKLFTGPSSTFVVQPQNGRCTTDGVLLGTTQLSPVNICTFRGGITRIGQSESYK
MNLASQNWNNYDPTEEIPAPLGTPDFKGRIRGLLTQTTKGAGSTRGHKATVLTGGSDFTP
KLGTVRFDTKSIDFENNQNTKFTPVGVVQDGNNHDEPTQWVLPNYSGPDTHNVHLAPAVA
PTFPGEQLLFFRSTMPGCSGYPKMELDCLLPQEWVQHFYQEAAPAQTDVALLRFVNPDTG
RVLFECKLHKSGYVTVAHTGDHDLVIPPNGYFRFDSWVNQFYTLAPMGNGTGRRRAL
SEQ ID No: 33:ab:AEG79288IGII.4 Hu/GII.4/Hong Kong/CUB001/2010/CHN 
MKMASNDANPSDGSAANLVPEVNNEVMALEPVAGAALAAPVAGQQNVIDPWIRNNFVQAP
GGEFTVSPRNAPGEILWSAPLSPDLNPYLSHLARMYNSYAGGFEVQVILAGNAFTAGKII
FAAVPPNFPIEGLSPSQVTMFPHIIVDVRQLEPVLIPLPDVRNNFYHYNQTNEPTIKLIA
MLYTPLRANNAGEDVFTVSCRVLTRPSPDFDFIFLVPPTVESRTKPFTVPILTVEEMTNS
RFPIPLEKLFTGPSSSFVVQPQNGRCTTDGVLLGTTQLSPVNICTFRGDVTHIAGSRNYT
MNLASINWNNYDPTEEIPAPLGTPDFVGKIQGMLTQTTRGEGSTRAHRATVYTGSAPFTP
KLGSVQFTTDTDNDFDANQNTKFTPVGVIQDGDTAHRNEPQQWVLPSYSGRNVQNVHLAP
AVAPTFPGEQLLFFRSTMPGCSGYPNMDLDCLLPQEWVQHFYQEAAPAQSDVALLRFVNP
DTGRVLFECKLHKSGYVTVAHTGQHDLVIPPNGYFRFDSWVNQFYTLAPMGNGAGRRRAL
SEQ ID No: 34:gb:AQU14484|GII.4 3.10 
MKMASNDANPSDGSAANLVPEVNNEVMALEPVVGAAIAAPVAGQQNVIDPWIRNNFVQAP
GGEFTVSPRNAPGEILWSAPLGPDLNPYLSHLARMYNGYAGGFEVQVILAGNAFTAGKII
FAAVPPNFPTEGLSPSQVTMFPHIIVDVRQLEPVLIPLPDVRNNFYHYNQSNDSTIKLIA
MLYTPLRANNAGDDVFTVSCRVLTRPSPDFDFIFLVPPTVESRSKPFSVPVLTVEEMTNS
RFPATLDKLFTGPSSTFVVQPQNGRCMIDGVLLGTTQLSPVNICTFRGDVTHLRDSPIYT
MNLASPNWNNYDSTEEIPAPLGTPDFVGKIQGVLTQTTKGEGSTRGHRATVYVGSANYTP
KLGKVQFETNTTNDLYAHQNTKFTPVGVVQGGESAHRSEPQQWVLPGYSGRDTPNVHLAP
AVAPTFPGEQLLFFRSTIPGCGGHPNMDLDCLLPQEWVQHFYQEAAPAQSDVALLRFVNP
DTSRVLFECKLHKSGYVTVAHTGQHDLVIPPNGYFRFDSWVNQFYTLAPMGNGTGRKRAI
SEQ ID No: 35:gb:AQU14462|GII.4 2.8b 
MKMASSDANPSDGSAANLVPEVNNEVMALEPVVGAAIAAPVAGQQNVIDPWIRNNFVQAP
GGEFTVSPRNAPGEILWSAPLGPDLNPYLSHLARMYNGYAGGFEVQVILAGNAFTAGKII
FAAVPPTFPTEGLSPSQVTMFPHIIVDVRQLEPVLIPLPDVRNNFYHYNQSNDSTIKLIA
MLYTPLRANNPGDDVFTVSCRVLTRPSPDFDFIFLVPPTVESRTKPFSVPILTVGEMTNS
RFPINLERLFTGPSSANVVQPQNGRCTIDGELLGTTQLSSVNICTFRGDVTHIGSTHHWT
MNLASPNWNNYDPTEETPAPLGTPDFVGKIHGMLTQTTQGNGSTRGHRATVYVGSAEFTP
KLGKVQFKTETDHDLAIRQNTKFTPVGVIQESDHHRDEPQQWRLPNYSGANTFNVHLAPA
VAPNFPGEQLLFFRSTLPGCGGHPNMDLDCLLPQEWVQHFYQEAAPAQSDVALLRFVNPD
TSRVLFECKLHKSGYVTVAHTGQYDLVLPSNGYFRFDSWVNQFYTLAPMGNGTGRRRAL
SEQ ID No: 36:gb:AHH44895|GII.4 Hu/GII.4/patient_C/2010/USA 
MKMASNDASPSDGSAANLVPEVNNEVMALEPVVGAAIAAPVAGQQNVIDPWIRNNFVQAP
GGEFTVSPRNAPGEILWSAPLGPDLNPYLSHLARMYNGYAGGFEVQVILAGNAFTAGKII
FAAVPPNFPTEGLSPSQVTMFPHIVVDVRQLEPVLIPLPDVRNNFYHYNQSKDPTIKLIA
MLYTPLRANNAGEDVFTVSCRVLTRPSPDFDFIFLVPPTVESRSKPFTVPILTVEEMTNS
RFPIPLEKLFTGPSGAFVVQPQNGRCTTDGVLLGTTQLSPVNICTFRGDLTHFTGTSEYA
MNLASQNWDNYDPTEEIPAPLGAPDFVGKIRGMLTQTTRRDGSTRGHRATLSTGSAHFTP
KLGNIRFSTDTNNDFEAGQNTKFTPVGVFQEGDNHQNEPQQWVLPNYSGATAHNVHLAPA
VAPAFPGEQLLFFRSTMPGCGGYPNMNLDCLLPQEWVQHFYQEAAPAQSDVALLRFVNPD
TSRVLFECKLHKSGYVTVAHTGQHDLVIPPNGYFRFDSWVNQFYTLAPMGNGTGRRRAL
SEQ ID No: 37:gb:AGX85889|GII.4 Hu/GII.4/NIHIC1.8/2012/USA 
MKMASSDANPSDGSTANLVPEANNEVMALEPVVGAAIAAPVAGQQNVIDPWIRNNFVQAP
GGEFTVSPRNAPGEILWSAPLGPDLNPYLSHLSRMYNGYAGGFEVQVILAGNAFAAGKII
FAAVPPNFPTEGLSPSQVTMFPHLIVDVRQLEPVMIPLPDIRNNFYHYNQSNDPTIKLIA
MLYTPLRANNVGDDVFTVSCRVLTRPSPDFDFIFLVPPTVESKTNPFSVPILTIEEMTNS
RFPIPLEKLFTGPSSALVVQPQNGRCTTDGVLLGTTQLSPVNICTFRGDVTHTTKTHTYQ
MNLAAQNWNSYDPTEEIPAPLGTPDFVGKIQGVLTQTTKGNGSTRAHKATVYTGSAEFTP
KLGRIQLFTDTDNDLEANQNTKFTPVGVIQDGDTHQNEPQQWVLPSYSGRNNHNVHLAPA
VAPTFPGEQLLFFRSTMPGCSGYPNMNLDCLLPQEWVQYFYQEAAPAQSDVALLRFVNPD
TGRVLFECKLHKSGYVTVAHTGHHDLFIPPNGYFRFDSWVSPFYTLAPMGNGTGRRRAL
SEQ ID No: 38:gb:AHH44878|GII.4 Hu/GII.4/patient A/2010/USA 
MKMASSDANPSDGSAANLVPEVNNEVMALEPVVGAAIAAPVAGQQNVIDPWIRNNFVQAP
GGEFTISPRNAPGEILWSAPLGPDLNPYLSHLARMYNGYAGGFEVQVVLAGNAFTAGKII
FAAVPPNFPTEGLSPSQVTMFPHIIVDVRQLEPVLIPLPDVRNNFYHYNQSKDSTIKLIA
MLYTPLRANNAGDDVFTVSCRVLTRPSPDFDFIFLVPPTVESRTKPFSVPVLTVEEMTNS
RFPIPLEKLFTGPSSAFVVQPQNGRCTTDGVLLGTTQLSAVNICTFRGDVTHVAGDTFAM
NLASLNWNNYDPTEETPAPLGTPDFVGRIHGMLTQTTRSDGATRAHKATVSTGGADFTPK
LGSVRYSTDTSSDLEVRENTKFTPIGVLHSSGGHRAEPDQWRLPEYSGRNVHNVHLAPAV
APTFPGEQLLFFRSTMPGCGGYPNMDLDCLLPQEWVQHFYQEAAPAQSDVALLRFVNPDT
GRVLFECKLHKSGYVTVAHTGPHDLVIPPNGYFRFDSWVNQFYTLAPMGNGTGRRRAL
SEQ ID No: 39:gb:BAW33648|GII.4 Hu/GII/3-157/Tokyo/1994/JPN 
MKMASSDANPSDGSAANLVPEVNNEVMALEPVVGAAIAAPVAGQQNIIDPWIRNNFVQAP
GGEFTVSPRNAPGEILWSAPLGPDLNPYLSHLSRMYNGYAGGFEVQVILAGNAFTAGKVI
FAAVPPNFPTEGLSPSQVTMFPHIIVDVRQLEPVLIPLPDVRNNFYHYNQSHDSTLKLIA
VLYTPLRTNNAGDDVFTVSCRVLTRPSPDFDFIFLVPPTVESRTKPFTVPILTVEEMSNS
RFPIPLEKLYTGPSSAFVVQPQNGRCTTDGVLLGTTQLSAVNICNFRGDVTHIVGSHETT
MNLASQNWSNYDPTQKIPAPLGTPHFVGKIQGLLTQTTRAHGSTRAHKATVSTGSVHFTP
KLGSVQFTTDTNNDFQTGQNTKFTPVGVIQDGDHHQNEPQQWVLPNYSGRTGHNVHLAPA
VAPTFPGEQLLFFRSTMPGCSGYPNMNLDCLLPHEWVLHFYQEAAPAQSDVALLRFVNPD
TGRVLFECKLHKSGYITVAHTGPFDLGIPPNGYFRFDSWVNQFYTLAPMGNGTGRRRAL
SEQ ID No: 40:gb:ABD77588|GII.4 Hu/Beijing/CR2905/2004/CHN 
MKMASNDASPSDGSTANLVPEVNNEVMALEPVVGAAIAAPVAGQQNVIDPWIRNSSVQAP
GGEFTVSPRDAPGEILWSAPLGPDLNPYLSHLARMYNGYAGGFEVQVILAGNAFTAGKII
FAAAPPNFPTEGLSPSQVTMFPHIIVGVRQLEPVLIPLPDVRNNFYHYNQSNDSTIKLIA
MLYTPLRANNAGDDVFTVSCRVLTRPSPDFDFIFLVPPTVESRTKPFTVPILTVEEMTNS
RFPIPLENCSRVPAVPLSSNHKMQCTTDGVLLGTTQLSPVNICTFRGDVTHIPGTRTYRM
NLASQNWNNYDPTEEIPAPLGTPDFVGKIQGMLTQTTKGDGSTRGHKATVSTGSVDFTPK
LGSVQFATDTDNDFETGQNTRFTPVGVIQDGSSTHRNEPQQWVLPDYSGRTVHNVHLAPA
VAPTFPGEQLLFFRSTMPGCSGYPNMDLDCLLPQEWVQHFYQEAAPSQSDVALLRFVNPD
TGRVLFECKLHKAGYVTVAHTGQHDLVIPPNGYFRFDSWVNQFYTLAPMGNGAGRRRAL
SEQ ID No: 41:gb:AGE99612|GII.4 Hu/GII.4/KL45/1978/MYS 
MKMASSDANPSDGSSANLVPEVNNEVMALEPVVGAAIAAPVAGQQNIIDPWIRNNFVQAP
GGEFTVSPRNAPGEILWSAPLSPDLNPYLSHLSRMYNGYAGGFEVQVILAGNAFTAGKIV
FAAVPPNFPTEGLSPSQVTMFPHIIVDVRQLEPVLIPLPDVRNNFYHYNQANDSTLKLIA
MLYTPLRANNAGDDVFTVSCRVLTRPSPDFDFIFLVPPTVESRTKPFTVPVLTVEEMTNS
RFPIPLERLFTGPSTAFVVQPQNGRCTTDGVLLGTTQLSAVNICNFRGEVNHIAGTHDYT
MRLTSQNWNNYDPTEEIPAPLGTPDFVGRIQGVLTQTTRSDGSTRSHKATVSTGSVHFTP
KLGSVQFTTDTTNDLNAGQNTKFTPVGVEQTSGDHQSEPQQWTLPNYSGTPNHNVHLAPA
VAPTFPGEQLLFFRSTLPGCGGYPNMNLDCLLPQEWVLHFYQEAAPAQSDVALLRFVNPD
TGRVLFECKLHRSGFITVAHSGSHDLVIPPNGYFRFDSWVNQFYTLAPMGNGSGRRRVV
SEQ ID No: 42:gb:AII73753|GII.5 Hu/JP/2002/GII.P5 GII.5/Saitama/T52 
MKMASNDATPSNDGAAGLVPESNNEAMALEPVVGASLAAPVTGQTNIIDPWIRTNFVQAP
NGEFTVSPRNSPGEILVNLELGPELNPYLAHLARMYNGYAGGMEVQVMLAGNAFTAGKII
FAAVPPYFPVENLSPSQITMFPHVIIDVRTLEPVLLPMPDVRSTLFHFNQKDEPKMRLVA
MLYTPLRSNGSGDDVFTVSCRILTRPSPEFDFTYLVPPTVESKTKPFTLPVLTLGELSNS
RFPLSIDEMVTSPNESIVVQPQNGRVTLDGELLGTTQLQACNICSIRGKVTGQVPNEQHM
WNLQITNLNGTQFDPTDDVPAPLGVPDFAGEVFGVLSQRNRGESNPANRAHDAVVATYSD
KYTPKLGLVQIGTWNTNDVENQPTKFTPIGLNEVANGHRFEQWTLPRYSGALTLNMNLAP
AVAPLFPGERLLFFRSYVPLKGGFGNPAIDCLVPQEWVQHFYQESAPSLGDVALVRYVNP
DTGRVLFEAKLHKGGFLTVSSTSTGPVVVPANGYFRFDSWVNQFYSLAPMGTGNGRRRFQ
SEQ ID No: 43:gb:BAN16287|GII.6 Hu/GII.6/Ehime090549/2009/JP 
MKMASNDAAPSNDGAANLVPEANNEVMALEPVAGASIAAPVVGQQNIIDPWIRENFVQAP
QGEFTVSPRNSPGEMLLNLELGPELNPYLSHLSRMYNGYAGGMQVQVVLAGNAFTAGKII
FAAVPPHFPVKNISAAQITMCPHVIVDVRQLEPVLLPLPDIRNRFFHYNQENTPRMRLVA
MLYTPLRANSGEDVFTVSCRVLTRPAPDFEFTFLVPPTVESKTKPFTLPILTLGELSNSR
FPAPIDMLYTDPNEGIVVQPQNGRCTLDGTLQGTTQLVPTQICAFRGTLIGQTSRSSDST
DSAPRRRDHPLHVQLKNLDGTQYDPTDEVPAVLGAIDFKGTVFGVASQRDVSGQQVGATR
AHEVHINTTDPRYTPKLGSILIHSESDDFVTGQPVRFTPIGMGDNDWHQWELPDYSGHLT
LNMNLAPAVAPAFPGERILFFRSMVPSAGGYGSGQIDCLIPQEWVQHFYQEAAPSQSAVA
LIRYVNPDTGRNIFEAKLHREGFITVANSGNNPIVVPHNGYFRFEAWVNQFYTLTPMGTG
QGRRRNQ
SEQ ID No: 44:gb:AII73774|GII.7 Hu/JP/2010/GII.P7_GII.7/Musashimurayama 
MKMASNDAAPSNDGAAGLVPEINNEVMPLEPVAGASLATPVVGQQNIIDPWIRNNFVQAP
AGEFTVSPRNSPGEILLDLELGPELNPYLAHLARMYNGHAGGMEVQIVLAGNAFTAGKII
FAAIPPGFPYENLSPSQITMCPHVIIDVRQLEPVLLPMPDIRNNFFHYNQGNDPKLRLIA
MLYTPLRANNSGDDVFTVSCRVLTKPSPDFEFTFLVPPTVESKTKQFTLPILKISEMTNS
RFPVPVEMMYTARNENQVVQPQNGRVTLDGELLGTTPLLAVNICKFKGEVIAKNGDVRSY
RMDMEITNTDGTPIDPTEDTPGPIGSPDFQGILFGVASQRNKNEQNPATRAHEANINTGG
DQYAPKLAQVKFFSESQDFEVHQPTVFTPVGVAGDTSHPFRQWVLPRYGGHLTNNTHLAP
AVAPLFPGEQILFFRSQIPSSGGHELGYMDCLVPQEWVQHFYQEAATAQSEVALIRFINP
DTGRVLFEAKLHKQGFITVAHTGDNPIVMPPNGYFRFEAWVNQFYSLAPVGTGNGRRRIQ
SEQ ID No: 45:qb:ATI15126|GII.8 Hu/JC231/ZS/GD/CHN/2016 
MKMASNDAAPSNDGAAGLVPEINHEVMAIEPVAGASLAAPVVGQLNIIDPWIRNNFVQAP
AGEFTVSPRNAPGEFLLDLELGPELNPYLAHLARMYNGHAGGMEVQIVLAGNAFTAGKIL
FAVIPPGFPYENLSPAQLTMCPHVVVDVRQLEPILLPMPDIRNTFFHYNQSNGPKLRLVA
MLYTPLRANNAGDDVFTVSCRVLTRPSPDFEFNFLVPPSVESKTKAFTLPILKISEMTNS
RFPIPVDQMYTSRNENVVVQPQNGRVTLDGELQGTTTLQPVSICGFRGTLQTRLADQPNY
TYQVHLENLDGSPVDPTDEVPAPLGTPDFQAQLFGVVSQRSSDNATRAHEARVNTNDPTF
APQIAQVRFKSPSHDFFDNEPIKFTPVGISVDSENSYNQWLLPRYGGHLTNNTHLAPSVS
PMFPGEQILFFRSFMPGASGHTDGAIDCLLPQEWVAHFYQEAATAQTDVALIRFVNPDTG
RVLFEGKLHKQGFITISNSGDHPIVMPANGYFRFEAWVNQFYSLAPVGTGSGRRRIQ
SEQ ID No: 46:gb:AGT39194|GII.12 Hu/GII.12/CGMH39/2010/TW 
MKMASNDAAPSNDGAAGLVPEVNNETMALEPVAGASIAAPLTGQNNVIDPWIRLNFVQAP
NGEFTVSPRNSPGEVLLNLELGPELNPYLAHLSRMYNGYAGGVEVQVLLAGNAFTAGKLV
FAAVPPHFPLENISPGQITMFPHVIIDVRTLEPVLLPLPDVRNNFFHYNQQNEPRMRLVA
MLYTPLRSNGSGDDVFTVSCRVLTRPSPDFDFNYLVPPTVESKTKPFTLPILTIGELTNS
RFPVPIDELYTSPNESLVVQPQNGRCALDGELQGTTQLLPTAICSFRGRINQKVSGENHV
WNMQITNINGTPFDPTEDVPAPLGTPDFSGKLFGVLSQRDHDNACRSHDAVIATNSAKFT
PKLGAIQIGTWEQDDVHINQPTKFTPVGLFESEGFNQWTLPNYSGALTLNMGLAPPVAPT
 
FPGEQILFFRSHIPLKGGVADPVIDCLLPQEWIQHLYQESAPSQTDVALIRFTNPDTGRV
LFEAKLHRSGYITVANTGSRPIVVPANGYFRFDSWVNQFYSLAPMGTGNGRRRVQ
SEQ ID No: 47:gb:BAQ94581|GII.13 Hu/GII.13/10N4555/2010/NP 
MKMASNDAAPSNDGAASLVPEAINETMPLEPVAGASIAAPVAGQTNIIDPWIRTNFVQAP
NGEFTVSPRNSPGEILLNLELGPDLNPYLAHLSRMYNGYAGGVEVQVLLAGNAFTAGKIL
FAAIPPNFPVDMISPAQITMLPHLIVDVRTLEPIMIPLPDVRNVFYHFNNQPQPRMRLVA
MLYTPLRSNGSGDDVFTVSCRVLTRPTPDFEFIYLVPPSVESKTKPFTLPILTISELTNS
RFPIPIEQLYTAPNENNVVQCQNGRCTLDGELQGTTQLLSSAVCSYRGRTVANRGDNWDQ
NLLQLTYPSGASYDPTDEVPAPLGTQDFSGILYGVLTQDNVSEGTGEAKNAKGVYISTTS
GKFTPKIGSIGLHSITENVHPNQQSRFTPVGVAQNENTPFQQWVLPHYAGALALNTNLAP
AVAPTFPGEQLLFFRSRVPCVQGLRGQDAFIDCLLPQEWVNHFYQEAAPSQADVALIRYV
NPDTGRTLFEAKLHRSGFITVSHTGAYPLVVPPNGHFRFDSWVNQFYS
SEQ ID No: 48:gb:AAN05735|GII.14 Hu/NLV/M7/1999/US 
MKMASNDATPSDDGAAGLVPEINNEVMALEPVAGASIAAPVVGQQNIIDPWIRNNFVQAP
AGEFTVSPRNSPGELLLDLELGPELNPYLAHLARMYNGHAGGMEVQIVLAGNAFTAGKIL
FAAIPPSFPYENLSPAQLTMCPHVIVDVRQLEPVLLPMPDIRNVFYHYNQNNSPKLRLVA
MLYTPLRANNSGDDVFTVSCRVLTRPSPDFQFTFLVPPTVESKTKNFTLPVLRVSEMTNS
RFPVVLDQMYTSRNENIIVQPQNGRCTTDGELLGTTTLQSVSICNFRGTMQAKLNEQPRY
QLQLTNLDGSPIDPTDDMPAPLGTPDFQAMLYGVASQRSSRDNATRAHDAQIDTAGDTFA
PKIGQVRFKSSSDDFDLHDPTKFTPIGVNVDDQHPFRQWSLPNYGGHLALNNHLAPAVTP
LFPGEQILFFRSHIPSAGGHTDGAIDCLLPQEWIEHFYQEAAPSQSDIALVRFINPDTGR
VLLEAKLHKQGFLTVAASGDHPIVMPTNGYFRFEAWVNPFYTLAPVGTGSGRRRIQ
SEQ ID No: 49:gb:AOQ30449|GII.17 E11161 
MKMASNDAAPSNDGAAGLVPEGNNETLPLEPVAGAAIAAPVTGQNNIIDPWIRTNFVQAP
NGEFTVSPRNSPGEILLNLELGPDLNPYLAHLSRMYNGYAGGVEVQVLLAGNAFTAGKIL
FAAVPPNFPVEFLSPAQITMLPHIIVDVRTLEPIMIPLPDVRNTFFHYNNQPNSRMRLVA
MLYTPLRSNGSGDDVFTVSCRVLTRPTPDFEFTYLVPPSVESKTKPFSLPILTLSELTNS
RFPVPIDSLFTAQNNVLQVQCQNGRCTLDGELQGTTQLLPSGICAFRGRVTAETDNPDKW
HMQLQNLNGTTYDPTDDVPAPLGTPDFKGVVFGVASQRNVGNDAPGSTRAHEAVISTYSP
KFVPKLGSVNFRSNDDDFQLQPTRFTPVGINDDGNHPFRQWELPDYSGVLTLNMNLAPPV
APNFPGEQLLLFRSFVPCSGGYNQGIIDCLIPQEWIQHFYQESAPSQSDVALIRYVNPDT
GRTLFEAKLHRSGYITVAHSGDYPLVVPANGYFRFDSWVNQFYSLAPMGTGNGRRRAQ
SEQ ID No: 50:gb:AII73747|GII.17 Hu/JP/2002/GII.P16 GII.17/Saitama/T87 
MKMASNDAAPSNDGATGLVPEINNETLPLEPVAGAAIAAPVTGQNNIIDPWIRTNFVQAP
NGEFTVSPRNSPGEILLNLELGPDLNPYLAHLSRMYNGYAGGVEVQVLLAGNAFTAGKIL
FAAVPPNFPVEFLSPAQITMLPHLIVDVRTLEPIMIPLPDVRNTFFHYNNQPANRMRLVA
MLYTPLRSNGSGDDVFTVSCRVLTRPTPDFEFTYLVPPSVESKTKPFSLPILTISELTNS
RFPAPIDSLFTAQNNNLNVQCQNGRCTLDGELQGTTQLLPSGICAFRGRLTADVDGSHDD
RWHMQLTNLNGTPFDPTEDVPAPLGTPDFTGLLFGVASQRNVGSNPNTTRAHEAVISTTS
SQFVPKLGSVNFGSTSTDFQLQQPTKFTPVGIKIESGHEFDQWALPRYSGHLTLNMNLAP
PIAPNFPGEQLLFFRSNVPCAGGVSDGVIDCLLPQEWIQHFYQESAPSQSDVALIRYVNP
DTGRTLFEAKLHRTGYITVAHSGDYPLVVPSNGYFRFDSWVNQFYSLAPMGTGNGRRRVQ
SEQ ID No: 51:gb:ALP48670|GII.21 Hu/GII.21/CUHK-NS-290/HKG/2014 
MKMASNDAAPSNDGAAGLVPEINTETLPLEPVAGAAIAAPVTGQNNIIDPWIRNNFVQAP
NGEFTVSPRNSPGEILMNLELGPDLNPYLAHLSRMYNGYAGGVEVQVLLAGNAFTAGKIL
FAAVPPNFPVDMLSPAQITMLPHLIVDVRTLEPIMIPLPDVRNVFYHFNNQPAPRMRLVA
MLYTPLRSNGSGDDVFTVSCRVLTRPTPDFEFTYLVPPSVESKTKPFTLPILTIGELTNS
RFPAPIDQLYTSPNADVVVQPQNGRCTLDGELQGTTQLLTTAICSYRGTTSNPTSDYWDD
HLLHLVHPNGATYDPTEDVPAPFGTQDFRGILYGVLTQNTQNPRDEVSNSRGIYISSTSD
 
KFTPKLGTIGLHQVQGDTASNQQSKFTPVGIAVNQNTPFKQWELPNYSGALTLNTNLAPA
VGPNFPGEQILFFRSNVPSVQGNHPTQEIDCLIPQEWISHFYQESAPSQSDVALVRYVNP
DTGRTIFEAKLHRQGFITIAATGSNPVVVPPNGYFRFDSWVNQFYALAPMGTGNGRRRAQ
SEQ ID No: 52:gb:AUD54981|GII.22 Hu/BD/2012/GII.P22-GII.22/Dhakal940 
MKMASNDAAPSNDGAAGLVPEINTEVMALEPVAGGAIAAPLTGQTNIIDPWIRNNFVQAP
NGEFTISPRNSPGEILLNMELGPELNPFLAHLSRMYNGFAGGMEVQVLMAGNAFTAGKVI
FAAIPPHFPVENLSPPQITMFPHIIIDVRTLEPVLLPMPDVRNQFFHYNQVNEPRMRLVA
MLYTPLRSNGSTEDVFTVSCRVLTRPSPDFEFNYLVPPTVESRTKPFTLPILTIGEMTNS
RFPAPIDMLYTSPNDNVVVQPQNGRCTLDGELQGSTQLVPANVCAFKGKITARIVDQAAH
QWHMQIDNPNGTLFDPTEDVPAPLGTPDFKAKIFGVISQRNDYNDGSQGPANRAHDAVVP
TTSAKFTPKLGSILVGTWENNDIETQPSKFTPVGLLEMNDFNQWSLPNYSGALTLNMGLA
PAVFPTFPGEQILFFRSFIPLKGGHGNPAIDCLLPQEWIQHFYQESAPSQTSVALIRYVN
PDTGRVLFEGKLHRQGFITIAKSGDGPIVVPPNGYFRFDSWVNQFYSLAPMGNGNGRRRI
Q
SEQ ID No: 53:gb:AUD54978|GII.24 Hu/PE/2014/GII.P24-GII.24/Loreto6424 
MKMASNDAAPSNDGAANLVPEANKEVMALEPVAGGAIAAPLTGQTNIIDPWIMNNFVQAP
NGEFTISPRNSPGEVLLNLELGPDLNPFLAHLSRMYNGYAGGVEVQVIMAGNAFTAGKVI
FAAVPPHFPVDNLSPPQVTMFPHVIVDVRTFEPILLPLPDVRNSFYHYNQVNDSRMRLIA
MLYTPLRSNGSSDDVFTVSCRVLTRPTPDFEFNYLVPPTVESRTKPFSVPILTIGEMTNS
RFPLPIDMLYTSPTENIVVQPQNGRCTLEGELLGTTQLVTPNICALRGEIRGHEGSGDNH
KWHFMVRSPNGAAFDPTEDVPAPLGTPDFIGDVFGVLSQRNRNTDSGQSGPANRSHDAVV
STRDSRFTPKLGSVMIATWETSDIQDQPTRFTPVGLENPDHYNQWQLPNYSGALTLNMGL
APSVFPTYPGEQILFFRSYIPLKGGYGDSHIDCLVPQEWIQHFYQESAPSQTDVALIRYV
NPETGRVLFEAKLHRQGYITVARSGSSPINVPANGYFRFDSWVNQFYSLAPMGTGNGRRR
IQ
SEQ ID No: 54:gb:AUD54984|GII.25 Hu/BD/2012/GII.P22-GII.25/Dhaka1928 
MKMASNDAAPSNDGAAGLVPEINNEVMALEPVAGGAIAAPLTGQTNVIDPWIRTNFVQAP
NGEFTISPRNSPGEVLLNMELGPELNPFLGHLSRMYNGYAGGIEVQVLMAGNAFTAGKVI
FAAVPPHFPVENLSPPQVTMFPHIIVDVRTLEPILLPLPDVRNQFFHYSQVDEPKMRLVA
MLYTPLRSNGSAEDVFTVSCRVLTRPSPDFEFNYLVPPTVESRTKPFTVPILTIGEMSNS
RFPAPIDMLYTSPNDNQNVQPQNGRCTLDGELQGTTQLVPSGVCAFRGRITGHEGSEQNQ
WHMQLTNLNGTPFDPTEDIPAPLGTPDFKGEIFGFISQRNAQNDPGQSQPANRAHDAVVS
TRAPKFTPKLGSVMIGTWVNSDIENQPSKFTPVGLNSNENFRQWELPDYSGVLTLNMGLA
PVVHPTYPGEQILFFRSYIPLKGGHGNPAIDCLLPQEWIQHFYQESAPSQTDVALLRYVN
PDTGRVLFEAKLHRQGYITIAKSGDGPIVVPPNGYFRFDSWVNQFYSLAPMGNGNGRRRV
Q
SEQ ID No: 55:gb:YP_009237904|GIV.1 Hu/GIV.1/Lake Macquarie/NSW2680/2010/AU 
MKMASSDAAPSTDGAGNLVPESQQEVLPLAPVAGAALAAPVVGQTNIIDPWIKENFVQAP
QGEFTVSPKNSPGEILVNLELGPKLNPYLDHLSRMYNSYAGGIDVMVVLAGNAFTAGKVL
IAAIPPNFPVEGVSASQATQFPHVIIDVRTLDPVRLPLPDVRSTFFHYTNDTEPKMRLVI
WLYTPLRTNGSGDDSFTVSGRILTRPSQDFEFAFLIPPTVETKTTPFSVPGFSVQEMSNS
RWPAAISAMVVRGNEPQVVQFQNGRAHLDGMLLGTTPVSPNYIASYRGISTGNSRSASSE
ADERAVGSFDVWVRLQEPDGQPYDIFGKQPAPIGTPDFKAVIVGFAARPLTSGSYANEAY
VNTTASDYAPATGNMRFTVRNGGTGHISANKYWEFKSFGVEGERHTDIQYQEYELPDYSG
QVASNHNLAPPVAPRMPGESLLLFQSNMPVWDDGHGESTPKKIHCLLPQEFIGHFFDRQA
PSLGDAALLRYVNQETNRVLFECKLYRDGYITVAASSGLLDFPLDGFFRFDSWVSSFYIL
SPVGSGQGRRGRVRFQ
SEQ ID No: 56:gb:APA31973|GIV.3 Hu/USA/2016/GIV.3/WI7002 
MKMASNDAPPSSDGAGNLVPESHQEVLPLAPVAGAELAAPVVGQTNIIDPWIKENFVQAP
QGEFTVSPKNAPGEILVNLELGPNLNPYLEHLSRMYNAYAGGIEVEIILAGNAFTAGKIL
IAAVPPNFPVESVSASQATQFPHAIVDVRTLEPVRLPLPDVRSNFFHYTTKDEPKMRLVI
WLYTPLRTNGSGDDSFTVSGRLLTRPSMDFQFSFLVPPTVETKTVLFTVPGLTPQEMSNS
RWPAQISGMVVRGNEPQVVQFQNGRCHTDGTLLGTTTVSEQCIAGFVGTSTNTRSATGST
TETRTGDTDLWLRLEEPNGQPYDIFGDQPAPLGTPDFRAVIVGFASRPQTQGSYMNEAYV
NTVDSHFAPATGNTKIILRRGGTGHVGGGHLWKFRPFGVEGGEGRVSYQEYVLPNYSGAT
ASNHNLAPPVAPRMPGELLLLFESDMPVWDDGHGAAPAQKIHCLLPQQFITHFFDSQAPA
LAEAALLRYVHPDSSRVLFETKLYREGFMVVSAPTGRFDFPLDGYFRFDSWVNSFYVLSP
VGSGQGRRGRSKVV
SEQ ID NO: 57: Ala-Ala-Leu-Leu/Val-His-Tyr
SEQ ID NO: 58: Ala-Ala-Leu-Leu/Val-Arg/Lys-Tyr
SEQ ID NO: 59: Ala-Ala-Leu-Leu/Val-His-Tyr-Val/Leu/Ile-Asp
SEQ ID NO: 60: Ala-Ala-Leu-Leu/Val-Arg/Lys- Tyr-Val/Leu/lle-Asp
SEQ ID NO: 61: Ala-Ala-Leu-Leu/Val-His

## Claims

1. A norovirus genogroup I VP1 capsid protein, wherein the amino acid sequence of said protein has at the position corresponding to Arg472 in SEQ ID NO:3 an Arg or Lys residue.

2. A norovirus genogroup I VP1 capsid protein having an amino acid sequence wherein the amino acid sequence stretch Ala-Ala-Leu-Leu/Val-His-Tyr in the P domain is modified to Ala-Ala-Leu-Leu/Val-Arg/Lys-Tyr, wherein Leu/Val means either Leu or Val, and Arg/Lys means Arg or Lys.

3. The capsid protein according to claim 2, wherein the amino acid sequence stretch Ala-Ala-Leu-Leu/Val-His-Tyr-Val/Leu/lle-Asp in the P domain is modified to Ala-Ala-Leu-Leu/Val-Arg/Lys-Tyr-Val/Leu/lle-Asp, wherein Val/Leu/Ile means either Val or Leu or Ile.

4. The capsid protein according to any one of claims 1 to 3, wherein said capsid protein belongs to any of the following genotypes of norovirus genogroup I: GI.1, GI.2, GI.3, GI.4, GI.5, GI.6, GI.7, GI.8, or GI.9.

5. The capsid protein according to any one of claims 1 to 4, wherein said protein has an amino acid sequence as defined in any one of SEQ ID NOs: 6 to 21, except that the amino acid residue at the position corresponding to Arg472 in SEQ ID NO:3 is Arg or Lys; or said protein has an amino acid sequence as defined in any one of SEQ ID NOs: 3 to 5.

6. The capsid protein according to any one of claims 1 to 5, wherein
(i) the amino acid sequence of said protein consists of or comprises at least 500 contiguous amino acid residues of the amino acid sequence of any one of SEQ ID NOs: 6 to 21, except that the amino acid residue at the position corresponding to Arg472 in SEQ ID NO:3 is Arg or Lys; or
(ii) the amino acid sequence of said protein consists of or comprises at least 500 amino acid residues and has at least 80%, preferably at least 85%, more preferably at least 90% sequence identity over this length to a sequence segment of at least 500 contiguous amino acid residues of the amino acid sequence of any one of SEQ ID NOs: 6 to 21; or
(iii) the amino acid sequence of said protein has from 1 to 50, preferably from 1 to 40, more preferably from 1 to 30 deletions, substitutions, additions or insertions compared to a sequence segment of at least 500 contiguous amino acid residues of the amino acid sequence of any one of SEQ ID NOs: 6 to 21;
whereby in items (ii) or (iii) the amino acid residue at the position corresponding to Arg472 in SEQ ID NO:3 is Arg or Lys.

7. The capsid protein according to any one of claims 1 to 6, wherein
(i') the amino acid sequence of said protein consists of or comprises the amino acid sequence from residue 43 to residue 540 of SEQ ID NO:3, 4 or 5; or
(ii') the amino acid sequence of said protein has at least 80 %, preferably at least 85%, more preferably at least 90% sequence identity to the amino acid sequence from residue 43 to residue 540 of SEQ ID NO:3, 4 or 5; or
(iii') the amino acid sequence of said protein has from 1 to 50, preferably from 1 to 40, more preferably from 1 to 30 deletions, substitutions, additions or insertions compared to the amino acid sequence from residue 43 to residue 540 of SEQ ID NO:3, 4 or 5;
whereby in items (ii') or (iii') the amino acid residue at the position corresponding to Arg472 in SEQ ID NO:3 is Arg or Lys.

8. Nucleic acid molecule encoding the capsid protein according to any one of claims 1 to 7.

9. Virus-like particle (VLP) consisting of or comprising the capsid protein according to any one of claims 1 to 7.

10. An immunogenic composition comprising the capsid protein as defined in any one of claims 1 to 7 or the VLP of claim 9 and optionally a pharmaceutically acceptable carrier.

11. The immunogenic composition according to claim 10, further comprising a Norovirus genogroup II VP1 capsid protein, or further comprising a VLP consisting of or comprising a genogroup II VP1 capsid protein.

12. A vaccine against norovirus infection, comprising the immunogenic composition of claim 10 or 11, or comprising the capsid protein as defined in any one of claims 1 to 7, or comprising the VLP of claim 9.

13. The vaccine according to claim 12, comprising a Norovirus genogroup II VP1 capsid protein or a VLP consisting of or comprising a genogroup II VP1 capsid protein.

14. The capsid protein as defined in any one of claims 1 to 7, or the VLP of claim 9, or the vaccine according to any one of claims 12 or 13 for use in the prevention or treatment of Norovirus infection in a subject, preferably a human subject.

15. A method of increasing the stability of Norovirus genogroup I VLPs, comprising replacing the His residue in the sequence stretch Ala-Ala-Leu-Leu/Val-His-Tyr in the P domain of a genogroup I VP1 capsid protein to Arg or Lys, preferably Arg.

## Patentansprüche

1. Norovirus-Genogruppe I VP1-Kapsidprotein, wobei die Aminosäuresequenz des Proteins an der Position, die Arg472 in SEQ ID NO:3 entspricht, einen Arg- oder Lys-Rest aufweist.

2. Norovirus-Genogruppe I VP1-Kapsidprotein mit einer Aminosäuresequenz, wobei der Aminosäuresequenzabschnitt Ala-Ala-Leu-Leu/Val-His-Tyr in der P-Domäne zu Ala-Ala-Leu-Leu/Val-Arg/Lys-Tyr modifiziert ist, wobei Leu/Val entweder Leu oder Val bedeutet und Arg/Lys Arg oder Lys bedeutet.

3. Das Kapsidprotein gemäß Anspruch 2, wobei der Aminosäuresequenzabschnitt Ala-Ala-Leu-Leu/Val-His-Tyr-Val/Leu/lle-Asp in der P-Domäne zu Ala-Ala-Leu-Leu/Val-Arg/Lys-Tyr-Val/Leu/lle-Asp modifiziert ist, wobei Val/Leu/lle entweder Val oder Leu oder Ile bedeutet.

4. Das Kapsidprotein gemäß einem der Ansprüche 1 bis 3, wobei das Kapsidprotein zu einem der folgenden Genotypen der Norovirus-Genogruppe I gehört: GI.1, GI.2, GI3, GI.4, GI5, GI.6, Gl.7, GI.8 oder Gl.9.

5. Das Kapsidprotein gemäß einem der Ansprüche 1 bis 4, wobei das Protein eine Aminosäuresequenz aufweist, wie sie in einer der SEQ ID NOs: 6 bis 21 definiert ist, mit der Ausnahme, dass der Aminosäurerest an der Position, die Arg472 in SEQ ID NO: 3 entspricht, Arg oder Lys ist; oder das Protein eine Aminosäuresequenz aufweist, wie sie in einer der SEQ ID NOs: 3 bis 5 definiert ist.

6. Das Kapsidprotein gemäß einem der Ansprüche 1 bis 5, wobei
(i) die Aminosäuresequenz des Proteins aus mindestens 500 benachbarten Aminosäureresten der Aminosäuresequenz einer der SEQ ID NOs: 6 bis 21 besteht oder diese umfasst, mit der Ausnahme, dass der Aminosäurerest an der Position, die Arg472 in SEQ ID NO: 3 entspricht, Arg oder Lys ist; oder
(ii) die Aminosäuresequenz des Proteins aus mindestens 500 Aminosäureresten besteht oder diese umfasst und mindestens 80 %, vorzugsweise mindestens 85 %, noch bevorzugter mindestens 90 % Sequenzidentität über diese Länge mit einem Sequenzsegment von mindestens 500 benachbarten Aminosäureresten der Aminosäuresequenz einer der SEQ ID NOs: 6 bis 21 aufweist; oder
(iii) die Aminosäuresequenz des Proteins 1 bis 50, vorzugsweise 1 bis 40, besonders bevorzugt 1 bis 30 Deletionen, Substitutionen, Additionen oder Insertionen im Vergleich zu einem Sequenzsegment von mindestens 500 benachbarten Aminosäureresten der Aminosäuresequenz einer der SEQ ID NOs: 6 bis 21 aufweist;
wobei in den Punkten (ii) oder (iii) der Aminosäurerest an der Position, die Arg472 in SEQ ID NO: 3 entspricht, Arg oder Lys ist.

7. Das Kapsidprotein gemäß einem der Ansprüche 1 bis 6, wobei
(i') die Aminosäuresequenz des Proteins aus der Aminosäuresequenz von Rest 43 bis Rest 540 von SEQ ID NO:3, 4 oder 5 besteht oder diese umfasst; oder
(ii') die Aminosäuresequenz des Proteins mindestens 80 %, vorzugsweise mindestens 85 %, noch bevorzugter mindestens 90 % Sequenzidentität mit der Aminosäuresequenz von Rest 43 bis Rest 540 von SEQ ID NO:3, 4 oder 5 aufweist; oder
(iii') die Aminosäuresequenz des Proteins 1 bis 50, vorzugsweise 1 bis 40, besonders bevorzugt 1 bis 30 Deletionen, Substitutionen, Additionen oder Insertionen im Vergleich zu der Aminosäuresequenz von Rest 43 bis Rest 540 von SEQ ID NO:3, 4 oder 5 aufweist;
wobei in den Punkten (ii') oder (iii') der Aminosäurerest an der Position, die Arg472 in SEQ ID NO: 3 entspricht, Arg oder Lys ist.

8. Nukleinsäuremolekül, das für das Kapsidprotein nach einem der Ansprüche 1 bis 7 kodiert.

9. Virusähnliches Partikel (VLP), das aus dem Kapsidprotein nach einem der Ansprüche 1 bis 7 besteht oder dieses enthält.

10. Immunogene Zusammensetzung, umfassend das Kapsidprotein nach einem der Ansprüche 1 bis 7 oder das VLP nach Anspruch 9 und gegebenenfalls einen pharmazeutisch akzeptablen Träger.

11. Die immunogene Zusammensetzung nach Anspruch 10, die ferner ein Norovirus-Genogruppe-II-VP1-Kapsidprotein oder die ferner ein VLP umfasst, das aus einem Genogruppe-II-VP1-Kapsidprotein besteht oder dieses umfasst.

12. Impfstoff gegen Norovirus-Infektionen, umfassend die immunogene Zusammensetzung nach Anspruch 10 oder 11 oder umfassend das Kapsidprotein, wie in einem der Ansprüche 1 bis 7 definiert oder umfassend das VLP nach Anspruch 9.

13. Impfstoff gemäß Anspruch 12, umfassend ein Norovirus-Genogruppe II VP1-Kapsidprotein oder ein VLP, das aus einem Genogruppe II VP1-Kapsidprotein besteht oder dieses umfasst.

14. Das Kapsidprotein gemäß einem der Ansprüche 1 bis 7 oder das VLP nach Anspruch 9 oder der Impfstoff nach einem der Ansprüche 12 oder 13 zur Verwendung bei der Vorbeugung oder Behandlung von Norovirus-Infektionen in einem Subjekt, vorzugsweise einem menschlichen Subjekt.

15. Verfahren zur Erhöhung der Stabilität von Norovirus-Genogruppe I-VLPs, umfassend das Ersetzen des His-Restes im Sequenzabschnitt Ala-Ala-Leu-Leu/Val-His-Tyr in der P-Domäne eines Genogruppe I VP1-Kapsidproteins durch Arg oder Lys, vorzugsweise Arg.

## Revendications

1. Protéine de capside VP1 du génogroupe I de norovirus, dans laquelle la séquence d'acides aminés de ladite protéine a, à la position correspondant à Arg472 dans la SEQ ID NO : 3, un résidu Arg ou Lys.

2. Protéine de capside VP1 du génogroupe I de norovirus ayant une séquence d'acides aminés dans laquelle l'étirement de séquence d'acides aminés Ala-Ala-Leu-Leu/Val-His-Tyr dans le domaine P est modifié en Ala-Ala-Leu-Leu/Val-Arg/Lys-Tyr, dans laquelle Leu/Val signifie soit Leu soit Val, et Arg/Lys signifie Arg ou Lys.

3. Protéine de capside selon la revendication 2, dans laquelle l'étirement de séquence d'acides aminés Ala-Ala-Leu-Leu/Val-His-Tyr-Val/Leu/Ile-Asp dans le domaine P est modifié en Ala-Ala-Leu-Leu/Val-Arg/Lys-Tyr-Val/Leu/Ile-Asp, dans laquelle Val/Leu/Ile signifie soit Val soit Leu soit Ile.

4. Protéine de capside selon l'une quelconque des revendications 1 à 3, dans laquelle ladite protéine de capside appartient à l'un quelconque des génotypes suivants de génogroupe I de norovirus : GI.1, GI.2, GI.3, GI.4, GI.5, GI.6, GI.7, GI.8 ou GI.9.

5. Protéine de capside selon l'une quelconque des revendications 1 à 4, dans laquelle ladite protéine a une séquence d'acides aminés telle que définie dans l'une quelconque des SEQ ID NO : 6 à 21, excepté que le résidu d'acide aminé à la position correspondant à Arg472 dans la SEQ ID NO : 3 est Arg ou Lys ; ou ladite protéine a une séquence d'acides aminés telle que définie dans l'une quelconque des SEQ ID NO : 3 à 5.

6. Protéine de capside selon l'une quelconque des revendications 1 à 5, dans laquelle
(i) la séquence d'acides aminés de ladite protéine consiste en ou comprend au moins 500 résidus d'acides aminés contigus de la séquence d'acides aminés de l'une quelconque des SEQ ID NO : 6 à 21, excepté que le résidu d'acide aminé à la position correspondant à Arg472 dans la SEQ ID NO : 3 est Arg ou Lys ; ou
(ii) la séquence d'acides aminés de ladite protéine consiste en ou comprend au moins 500 résidus d'acides aminés et a une identité de séquence d'au moins 80 %, de préférence d'au moins 85 %, plus préférablement d'au moins 90 %, sur sa longueur, avec un segment de séquence d'au moins 500 résidus d'acides aminés contigus de la séquence d'acides aminés de l'une quelconque des SEQ ID NO : 6 à 21 ; ou
(iii) la séquence d'acides aminés de ladite protéine a de 1 à 50, de préférence de 1 à 40, plus préférablement de 1 à 30 délétions, substitutions, additions ou insertions en comparaison avec un segment de séquence d'au moins 500 résidus d'acides aminés contigus de la séquence d'acides aminés de l'une quelconque des SEQ ID NO : 6 à 21 ;
moyennant quoi, dans les points (ii) ou (iii), le résidu d'acide aminé à la position correspondant à Arg472 dans la SEQ ID NO : 3 est Arg ou Lys.

7. Protéine de capside selon l'une quelconque des revendications 1 à 6, dans laquelle
(i') la séquence d'acides aminés de ladite protéine consiste en ou comprend la séquence d'acides aminés allant du résidu 43 au résidu 540 de la SEQ ID NO : 3, 4 ou 5 ; ou
(ii') la séquence d'acides aminés de ladite protéine a une identité de séquence d'au moins 80 %, de préférence d'au moins 85 %, plus préférablement d'au moins 90 % avec la séquence d'acides aminés allant du résidu 43 au résidu 540 de la SEQ ID NO : 3, 4 ou 5 ; ou
(iii') la séquence d'acides aminés de ladite protéine a de 1 à 50, de préférence de 1 à 40, plus préférablement de 1 à 30 délétions, substitutions, additions ou insertions en comparaison avec la séquence d'acides aminés allant du résidu 43 au résidu 540 de la SEQ ID NO : 3, 4 ou 5 ;
moyennant quoi, dans les points (ii') ou (iii'), le résidu d'acide aminé à la position correspondant à Arg472 dans la SEQ ID NO : 3 est Arg ou Lys.

8. Molécule d'acide nucléique codant la protéine de capside selon l'une quelconque des revendications 1 à 7.

9. Particule pseudovirale (PPV) consistant en ou comprenant la protéine de capside selon l'une quelconque des revendications 1 à 7.

10. Composition immunogénique comprenant la protéine de capside telle que définie dans l'une quelconque des revendications 1 à 7 ou la PPV de la revendication 9 et optionnellement un véhicule pharmaceutiquement acceptable.

11. Composition immunogénique selon la revendication 10, comprenant en outre une protéine de capside VP1 de génogroupe II de norovirus, ou comprenant en outre une PPV consistant en ou comprenant une protéine de capside VP1 de génogroupe II.

12. Vaccin contre une infection par un norovirus, comprenant la composition immunogénique de la revendication 10 ou 11, ou comprenant la protéine de capside telle que définie dans l'une quelconque des revendications 1 à 7, ou comprenant la PPV de la revendication 9.

13. Vaccin selon la revendication 12, comprenant une protéine de capside VP1 de génogroupe II de norovirus ou une PPV consistant en ou comprenant une protéine de capside VP1 de génogroupe II.

14. Protéine de capside selon l'une quelconque des revendications 1 à 7, ou PPV selon la revendication 9, ou vaccin selon l'une quelconque des revendications 12 et 13, pour une utilisation dans la prévention ou le traitement d'une infection par un norovirus chez un sujet, de préférence un sujet humain.

15. Procédé pour augmenter la stabilité de PPV de génogroupe I de norovirus, comprenant le remplacement du résidu His dans l'étirement de séquence Ala-Ala-Leu-Leu/Val-His-Tyr dans le domaine P d'une protéine de capside VP1 de génogroupe I par Arg ou Lys, de préférence Arg.
